(19) Europäisches Patentamt
European Patent Office
Office européen des brevets

(11) EP 4 023 204 A1

(12) EUROPEAN PATENT APPLICATION

(43) Date of publication:
**06.07.2022 Bulletin 2022/27**

(21) Application number: **20397518.0**

(22) Date of filing: **30.12.2020**

(51) International Patent Classification (IPC):
***A61K 6/75*** (2020.01) ***A61K 6/816*** (2020.01)
***A61K 6/871*** (2020.01) ***A61K 6/873*** (2020.01)
***A61K 6/887*** (2020.01) ***C08L 33/10*** (2006.01)
***A61K 6/40*** (2020.01) ***A61K 6/824*** (2020.01)
***A61K 6/838*** (2020.01)

(52) Cooperative Patent Classification (CPC):
(C-Sets available)
**A61K 6/77; A61K 6/40; A61K 6/75; A61K 6/816; A61K 6/824; A61K 6/838; A61K 6/871; A61K 6/873; C08L 33/10** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Stick Tech OY**
**20521 Turku (FI)**

(72) Inventors:
- **LASSILA, Lippo**
  **21630 Lielax (FI)**
- **GAROUSHI, Sufyan**
  **20540 Turku (FI)**
- **VALLITTU, Pekka**
  **21620 Kuusisto (FI)**
- **SÄILYNOJA, Eija**
  **20660 Littoinen (FI)**

(74) Representative: **Laine IP Oy**
**Porkkalankatu 24**
**00180 Helsinki (FI)**

# (54) A KIT OF PARTS FOR DENTAL RESTORATION

(57) The present invention relates to a kit for dental restoration comprising an aqueous activation agent solution comprising polyacrylic acid having a molecular weight of 100000-1000000 g/mol, wherein the concentration of the polyacrylic acid in the solution is 1-30 mg/1; and a lining material comprising 20-80 wt-% of a dental resin and 20-80 wt-% of an ion releasing material, which ion releasing material comprises at least one of calcium, phosphate and zinc, based on the total weight of the lining material.

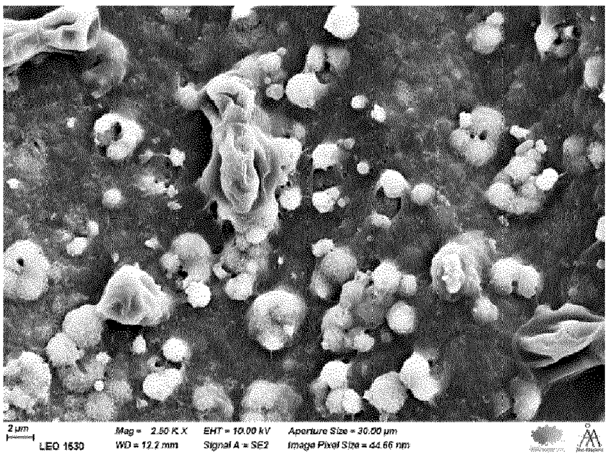


FIG. 5B

EP 4 023 204 A1

(52) Cooperative Patent Classification (CPC): (Cont.)

C-Sets
**A61K 6/887, C08L 33/10**

## Description

FIELD

The present invention relates to dental restorations and method for making dental restorations.

BACKGROUND AND OBJECTS

Tooth decay, also known as dental caries or cavities, is the breakdown of teeth due to acids made by bacteria. Caries is treated by removing the carious lesions and replacing them with restorative material. In the caries infected dentin, the organic matrix is irreversibly damaged, while the deeper caries affected dentin is hypomineralised with sound organic matrix, which could be repaired and remineralised.

Minimally invasive cavity preparation with manual removal of carious lesions by using dentin excavators is at the moment one of the recommended approaches to avoid generation of aerosol and droplet during restorative treatment. Minimally invasive approaches aim eventually to minimize the excavation of dental tissues and encourage their recovery and repair instead. The slow progression of caries indeed allows a reparative intervention, which can restore the mineralised architecture after excavating the infected layer.

For the remineralisation process, different approaches have been applied, which can be classified as classical and non-classical approaches. In the classical approach, dentin remineralisation is based on the epitaxial growth of residual crystallites, which act as nucleation sites for the calcium phosphate minerals to precipitate when dentin is stored in a solution rich with calcium and phosphate ions. However, recent studies have indicated that such an approach would result in an incomplete and non- functional remineralisation of dentin. The classical remineralisation approach results in extra-fibrillar remineralisation of the collagen matrix of dentin, without the mineralization of collagen's intra-fibrillar compartments. This is believed to be due to the size of the apatite crystals forming during this process, in the absence of any control on their size and orientation. Consequently, the non-classical approach has been suggested as an alternative in-vitro remineralisation technique, which attempted to achieve a hierarchical biomimetic remineralisation of the organic matrix of dentin.

To have a biomimetic non-classical remineralisation process, two things are needed. First, the use of synthetic substitutes for certain dentin matrix proteins that play an essential role during the biomineralisation process (formation and stabilisation of amorphous calcium phosphate). Second, a cavity liner to be used as a source for calcium and phosphate. Known solutions consist in treating a surface which has been cleaned of carious lesions, with a cavity liner, thereafter applying a restorative material.

An aim of the present invention is thus to develop an easy-to-use product for dental restorations, which also may enhance dentin remineralisation. Advantageously, the product would lead to a strong finished restoration. A further advantage would be that the product would be antimicrobial, i.e. it would limit bacterial growth.

SUMMARY OF THE INVENTION

The invention is defined by the features of the independent claim. Some specific embodiments are defined in the dependent claims. According to an aspect of the present invention, there is provided a kit for dental restoration comprising

- an aqueous activation agent solution comprising polyacrylic acid having a molecular weight of 100000-1000000 g/mol, wherein the concentration of the polyacrylic acid in the solution is 1-30 mg/l, and
- a lining material comprising 20-80 wt-% of a dental resin and 20-80 wt-% of an ion releasing material, which ion releasing material comprises at least one of calcium, phosphate and zinc, based on the total weight of the lining material.

BRIEF DESCRIPTION OF THE DRAWINGS

Figure 1 schematically illustrates a theory behind the present invention in accordance with an embodiment.
Figures 2A and 2B illustrate sample preparation.
Figures 3A-5B are SEM photos following immersion of samples in SBF at various points of time
Figures 6A and 6B are SEM photos of samples in Figures 5A and 5B, from a different angle.
Figures 7A and 7B are SEM photos of the interface between the dentin treated with the activation agent solution and the material according to the present invention as applied.

Figures 8-10 illustrate the major elemental composition of different parts of said interface.

Figures 11-14 are SEM photos of the interface between a commercial material and the dentin.

Figures 15-18 are SEM photos of the dentin surface that was cut under various conditions and immersed in SBF for one week.

Figure 19 is a cross-sectional SEM photo of the sample in Figure 18.

Figure 20 illustrates the major elemental composition of different parts of the interface of Figure 19.

Figure 21 illustrates the results of testing dissolution of calcium (g/kg) to water in 24 hours, from disc samples according to some embodiments.

Figure 22 illustrates the results of measurements of dissolution of zinc (g/kg) to water at three different moments of time, according to one embodiment.

Figure 23 illustrates the results of measurements of dissolution of calcium (g/kg) from samples to water at three different moments of time, according to another embodiment.

Figure 24 shows flexural strength in MPa of various comparative examples and of several samples prepared according to an embodiment.

Figure 25 illustrates the flexural strength in MPa of two samples according to some embodiments.

Figure 26 illustrates the flexural strengths in MPa for some comparative samples.

Figures 27A and 27B illustrate the test setting for measuring shear-bond strength.

Figure 28 shows the shear-bond strength in MPa for samples according to some further embodiments.

Figure 29 shows the shear-bond strength in MPa for samples according to yet some further embodiments.

Figures 30A-31B are SEM photos of dentin surfaces, following various treatments.

Figure 32 shows the fracture toughness of some samples.

DETAILED DESCRIPTION

In the present description, the term "antimicrobial" means that the material kills or inhibits the growth of microorganisms but causes little or no damage to the host. The term "remineralisation of dentin" stands for the natural repair process for non-cavitated tooth lesions, in which calcium, phosphate and sometimes fluoride ions are deposited into crystal voids in demineralised enamel. Remineralisation can contribute towards restoring strength and function within tooth structure. The term "release rate" means the amount of ions released from a sample within a given time. In this context, "a surface modifier" is a component or compound which primary aim is to activate the mineralisation in the tooth to be restored. The term "dental resin" stands for a monomer mixture or a polymer capable of polymerising and/or crosslinking, that is suitable for use in dentistry, i.e. that is biocompatible and has sufficient strength.

In the context of the present application an average length of fibres is determined as follows. The fibres are divided into a number of fractions with 0.1 mm intervals according to their length. The fibre lengths in each 0.1 mm interval are added together. The average fibre length is taken to be value where the lengths of the shorter fibres and longer fibres are deemed to be the same. The same determination method is used for determining an average size of particles, and the measured dimension is the largest dimension of the particle. Different measurement methods typically provide identical results, and one possible measurement method is disclosed in ISO 13320:2009.

According to an aspect of the present invention, there is provided a kit for dental restoration comprising

- an aqueous activation agent solution comprising polyacrylic acid having a molecular weight of 100000-1000000 g/mol, wherein the concentration of the polyacrylic acid in the solution is 1-30 mg/l, and
- a lining material comprising 20-80 wt-% of a dental resin and 20-80 wt-% of an ion releasing material, which ion releasing material comprises at least one of calcium, phosphate and zinc, based on the total weight of the lining material.

The kit thus provides a combination of a dentin activator solution and lining material serving as a strong cavity liner and containing additives that have remineralising (for example due to calcium and/or phosphate) and/or antimicrobial (for example due to zinc) capabilities. The ion releasing material(s) of the lining material is in a form capable of being released from the material, some examples being given below. In some cases, the lining material may also be in the form of a flowable composite, and preferably it is bioactive. Without wishing to be bound by any theory, it is believed that the activation agent solution makes the dentin surface, including collagen fibres, negatively charged, to attract the ions from the ion releasing material, and stabilise the formation of hydroxyapatite crystals. The combination of materials and their properties was selected based on mechanical and chemical properties as well as handling properties.

The aqueous activation agent solution (also called activation solution) is typically mostly or solely composed of water and polyacrylic acid (PAA). It is also possible to use another polyelectrolyte than polyacrylic acid, or to use further components in small amounts. For example, the activation agent solution may further comprise 10-methacryloyloxydecyl dihydrogen phosphate (MDP). In case it is used, it is typically used in a concentration of 0.8-7.5 mg/ml.

According to experimental data, concentrations of 0.4 and 8.3 mg/ml still have a small effect, but the range of 0.8-7.5 mg/ml is believed to be the optimum in the present context. The effect of MDP is that it increases the amount of initial bonds, thus improving the durability of bonded restorations. Further optionally, other well-known bonding adhesion promoters such as 4-methacryloyloxyethy trimellitate anhydride (4-META), N-(2-hydroxy-3-((2-methyl-1-oxo-2-propenyl) oxy) propyl)-N-tolyl glycine (NTG-GMA), bis-(-)-nor-meptazinol (BisMEP), bis(glyceryl dimethacrylate) pyromellitate (PMDGM) or 1,3-glycerol dimethacrylate/maleate (GDMA maleate), or any mixture thereof could be used instead of the MDP or in addition to it.

According to an embodiment, the concentration of MDP is 1.0-6.5 mg/ml. According to another embodiment, the concentration of MDP is 2.0-5.5 mg/ml. The concentration of MDP may however be for example from 0.8, 0.9, 1.0, 1.2, 1.5, 1.7, 2.0, 2.2, 2.5, 2.7, 3.0, 3.5, 4.0, 4.5, 5.0, 5.5, 6.0, 6.5 or 7.0 mg/ml up to 1.2, 1.5, 1.7, 2.0, 2.2, 2.5, 2.7, 3.0, 3.5, 4.0, 4.5, 5.0, 5.5, 6.0, 6.5, 7.0 or 7.5 mg/ml.

The molecular weight of PAA used in the activation agent solution is 100000-1000000 g/mol. According to an embodiment, the molecular weight of the polyacrylic acid (PAA) is 250000-600000 g/mol. It was found in experiments that a molecular weight of 10000 g/mol was too low and that a molecular weight of 4000000 g/mol was too high in the present context. The molecular weight of PAA may be for example from 100000, 120000, 150000, 200000, 250000, 300000, 350000, 400000, 450000, 500000, 550000, 600000, 650000, 700000, 750000, 800000, 850000 or 900000 g/mol up to 150000, 200000, 250000, 300000, 350000, 400000, 450000, 500000, 550000, 600000, 650000, 700000, 750000, 800000, 850000, 900000, 950000 or 1000000 g/mol.

The concentration of the polyacrylic acid in the solution is 1-30 mg/l. According to an embodiment, the concentration of PAA in the solution is 5-20 mg/l. The concentration may be for example from 1, 3, 5,7, 10, 12, 15, 18, 20, 22, 25 or 28 mg/l up to 3, 5,7, 10, 12, 15, 18, 20, 22, 25, 28 or 30 mg/l.

Both the selected molecular weight and the selected concentration of PAA are relevant for the end result, as will be demonstrated in the Experimental part below.

Typically, the activation agent solution is suitable for use as a drilling solution or as a surface modifier during dental restoration. Drilling solutions and surface modifiers as well their uses are known as such, and the present kit comprises the activation agent solution in a form suitable for either use. Typically, the viscosity of the drilling solution is somewhat lower than the viscosity of the surface modifier.

The kit comprises a lining material comprising 20-80 wt-% of a dental resin and 20-80 wt-% of an ion releasing material, based on the total weight of the lining material. The amount of the dental resin can be for example from 20, 25, 30, 35, 40, 45, 50, 55, 60, 65 or 70 wt-% up to 30, 35, 40, 45, 50, 55, 60, 65, 70, 75 or 80 wt-%. The amount of the ion releasing material can be for example from 20, 25, 30, 35, 40, 45, 50, 55, 60, 65 or 70 wt-% up to 30, 35, 40, 45, 50, 55, 60, 65, 70, 75 or 80 wt-%. Some examples of the different amounts are given below.

According to an embodiment, the ion releasing material comprises calcium and phosphate. According to another embodiment, the ion releasing material comprises calcium and zinc. According to yet another embodiment, the ion releasing material comprises phosphate and zinc. According to a preferred embodiment, the ion releasing material comprises calcium, phosphate and zinc. According to another preferred embodiment, the ion releasing material comprises calcium, phosphate, zinc and titanium. The ion releasing material may also comprise further materials, such as bioactive glass, aluminium and/or titanium. The ion releasing material may be selected from hydroxyapatite, carbonated apatite, calcium carbonate, bioactive glass, zinc oxide containing glass, aluminium calcium silicate glass, titanium oxide containing glass and mixtures thereof.

According to an embodiment, the composition of the ion releasing material is

- 35-55 wt-% of carbonated apatite,
- 2-10 wt-% of calcium carbonate, and
- 40-60 wt-% of ZnO containing glass,

based on the total weight of the ion releasing material.

According to another embodiment, the composition of the ion releasing material is

- 40-50 wt-% of carbonated apatite,
- 4-8 wt-% of calcium carbonate, and
- 45-55 wt-% of ZnO containing glass,

based on the total weight of the ion releasing material.

The release rate of the various ion(s) from the ion releasing material may be controlled in different ways. One way of controlling the release rate is the degree of crystallisation. Typically, if the ion releasing material is an apatite, carbonate and/or glass and highly crystallised (such as having a degree of crystallisation of 20 % or higher, or 70 % or higher, depending on the material), it may be rather inert, i.e. ions are released slowly. The suitable degree of crystallisation

for a desired release rate depends on the material selected, and can vary significantly. However, for most materials, for ions to be released quickly, the material should be amorphous. The release rate also depends on the pH of the environment. It is thus possible and advantageous to combine in the ion releasing material at least two different materials having different release rates. In this case, the material with high release rate releases its ions quickly, while the material with lower release rate releases its ions more slowly, and hence over a longer period of time.

The lining material is preferably suitable for use as a liner material or a base material during dental restoration. Again, the use of liner and base materials is known as such, as are the required properties such as viscosity and wetting. The difference between a liner material and a base material is known to a person skilled in the art and can be summarised as a liner material being applied on the bottom of the cavity to be restored, as a thin layer (typically 0.5-1 mm, or even thinner than 0.5 mm), while the base material is applied in a slightly thicker layer (typically 2-4 mm).

The lining material also comprises a dental resin, which is typically selected from a group consisting of methyl methacrylate, ethyl methacrylate, n-butyl methacrylate, isobutyl methacrylate, 2-ethylhexyl methacrylate, cyclohexyl methacrylate, isobornyl methacrylate, tetrahydrofurfuryl methacrylate, benzyl methacrylate, morpholinoethyl methacrylate, diethylene glycol dimethacrylate, triethylene glycol dimethacrylate, diurethane dimethacrylate, 2,2-bis(4-(2-hydroxy-3-methacryloxy)phenyl)propane, acrylic acid, epoxy, bisphenol A-glycidyl methacrylate (BisGMA), urethane dimethacrylate (UDMA), semi-crystalline polyceram (PEX), trimethylolpropane ethoxylate triacrylate and mixtures thereof.

The present kit may further comprise a dental restoration resin. The dental restoration resin is typically selected from a group consisting of methyl methacrylate, ethyl methacrylate, n-butyl methacrylate, isobutyl methacrylate, 2-ethylhexyl methacrylate, cyclohexyl methacrylate, isobornyl methacrylate, tetrahydrofurfuryl methacrylate, benzyl methacrylate, morpholinoethyl methacrylate, diethylene glycol dimethacrylate, triethylene glycol dimethacrylate, diurethane dimethacrylate, 2,2-bis(4-(2-hydroxy-3-methacryloxy)phenyl)propane, acrylic acid, epoxy, bisphenol A-glycidyl methacrylate (BisGMA), urethane dimethacrylate (UDMA), semi-crystalline polyceram (PEX), trimethylolpropane ethoxylate triacrylate and mixtures thereof. Thus, the dental restoration resin can be the same as the resin of the lining material, or it may be different from it. The dental restoration resin may also comprise fillers, such as any of the ones listed below.

The resin in the lining material and/or the dental restoration resin may also be a compound as disclosed in WO 2020/035321, the content of which is herein incorporated by reference, in particular in reference to the manufacturing of this compound. The compound can thus have a general formula (I)

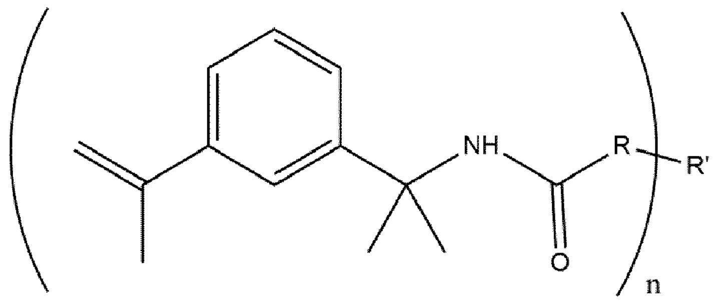

(I)

wherein when n=1, R=NH, R' is (Ia) or (Ib)

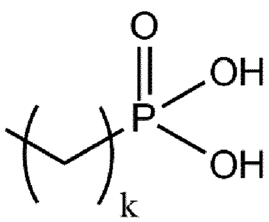

(Ia),

wherein k is 2 or 3,

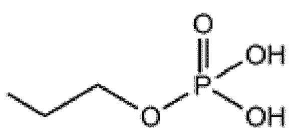

(Ib),

when n=2, R=O, R' is (Ic) or (Id)

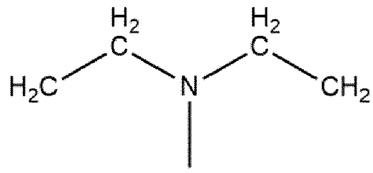

(Ic)

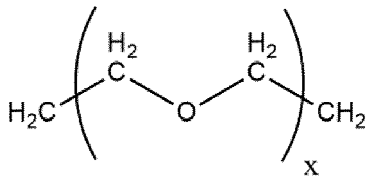

(Id),

wherein x=1-12,
when n=2, R=NH or O, R' is (Ie)

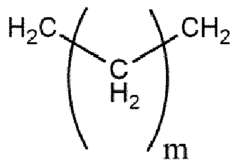

(Ie),

wherein m=0-16, and
when n=2, R=O, R' is (If)

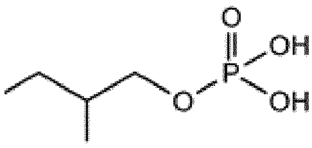

(If).

The lining material may still further comprise fillers. Such fillers may be for example bioactive or partially reactive glass ionomer fillers containing elements such as oxides of silicon (Si), calcium (Ca), phosphorus (P), barium (Ba), magnesium (Mg), potassium (K), titanium (Ti), fluorine (F), strontium (Sr), zinc (Zn), cerium (Ce), niobium (Nb) or other compounds of said elements, colour pigments, inert ceramics, inert silica, hydroxyl apatite (HA) or other Ca-phosphates, $Al_2O_3$, $ZrO_2$, Ag, zerogels, bioactive glasses or filler particles containing functional bioactive or therapeutically active molecules, antigens, antibiotics, disinfectants, radio-opaque materials, organic acids such as maleic acids, polyacrylic acid, or the like. Any of these fillers may also be incorporated into the dental restoration resin.

According to a preferred embodiment, the lining material still further comprises inert fillers. The fillers are typically selected from short E- glass fibres, S-glass fibres and mixtures thereof. Any of these fillers may also be incorporated into the dental restoration resin.

According to an embodiment, the relative amounts of the ion releasing material, the dental resin and the inert fillers in the lining material are

- 30-50 wt-% of the ion releasing material,
- 30-50 wt-% of the dental resin, and
- 10-30 wt-% of the inert fillers,

based on the total weight of the lining material.

According to another embodiment, the relative amounts of the ion releasing material, the dental resin and the inert fillers in the lining material are

- 35-45 wt-% of the ion releasing material,

- 35-45 wt-% of the dental resin, and
- 15-25 wt-% of the inert fillers,

based on the total weight of the lining material.

The fillers may be in particle form or in fibre form. By particles, it is meant also for example spheres and very short fibres (where the length of the fibre is at most two times its diameter), while being significantly shorter than the short fibres, like whiskers i.e. having a length below 50 μm. In case of fillers in the form of fibres, the length of the fibre may range from 80 μm to 300 μm (micrometre). More optionally, the average length of the fibres ranges from 100-300 μm. For example, the average length of the filler fibres may be from 100, 120, 150, 170, 200, 220, 230 or 250 μm up to 120, 150, 180, 200, 210, 220, 230, 250, 280 or 300 μm. Optionally, in a preferred embodiment, the average length of the fibres in the material ranges from 150-250 μm. The average largest dimension of the particulate fillers can be for example 0.3 - 25 μm. The diameter of the particles in the filler material is thus, in the case of irregular particles, the largest diameter of the particles. The diameter can be from 0.3, 0.5, 1, 5, 7, 10, 13, 15, 17, 20 or 22 μm up to 1, 5, 7, 10, 13, 15, 17, 20, 22 or 25 μm. These suitable dimensions also apply to the ion releasing materials above.

The average length of the fibres is determined by dividing the fibres into a number of fractions with 0.1 mm (millimetre) intervals according to their length. The fibre lengths in each 0.1 mm interval are added together. Moreover, the average fibre length is taken to be the value where the lengths of the shorter fibres and longer fibres are deemed to be the same. The same process is used for determining the average largest dimension of the particles.

When using the present kit, the dentist first prepares the cavity to be restored. During drilling, the activation solution can be used as a drilling solution (which may also be called rinsing solution). Alternatively, it may be applied as a surface modifier, i.e. applied on the inner surface of the cavity as a first step after drilling and rinsing (either on the whole inner surface or only the bottom of the cavity). Thereafter, the lining material is applied, either as a liner material or as a base material, followed by curing, typically light-curing, of the liner or base material. A third step is then the application of the dental restoration, typically a resin, and curing of the resin. The dental restoration thus covers the lining material, thereby preventing leaching of the ions to the patient's mouth.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Furthermore, the described features, structures, or characteristics may be combined in any suitable manner in one or more embodiments. In the description, numerous specific details are provided, such as examples of lengths, widths, shapes, etc., to provide a thorough understanding of embodiments of the invention.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", i.e. a singular form, throughout this document does not exclude a plurality.

DETAILED DESCRIPTION OF THE DRAWINGS

Figure 1 schematically illustrates a theory behind the present invention in accordance with an embodiment. In the Figure, the ion releasing material is schematically shown as the area 1, here as comprising various ions that can be released. The dentin (shown with reference number 2) has been treated with an activation solution, thus its surface attracts $Ca^{2+}$ ions (as one example of the ions). This difference in electrical charge is believed to induce the migration of the ions from the ion releasing material towards the dentin.

Figures 2A and 2B illustrate the sample preparation as is explained in more detail below. Figures 3A-32 illustrate the results of tests carried out on the samples and are explained in more detail below in connection with the Experimental part.

EXPERIMENTAL PART

Several different samples according to the present description were prepared, and compared to samples prepared using commercial products. Various measurements were made, as will be explained below.

**Sample preparation**

The activation agent solution was prepared by mixing distilled water (grade III) and polyacrylic acid (PAA) from Sigma-Aldrich (St. Louis, MO, USA) having a given molecular weight. Different molecular weights (5000 g/mol, 450000 g/mol and 4000000 g/mol) and concentrations (10 mg/, 20 mg/l, 2 g/l and 500 g/1) of the PAA in water were tested.

When 10-methacryloyloxydecyl dihydrogen phosphate (MDP, from Fluorochem Ltd, Hadfield, England) was used, its concentration was as given below in Table 2.

The lining material was prepared by mixing the components together. The following components were used:

- E-glass fibres having a diameter of 6 μm and a distribution of length 50-200 μm, from GC Dental
- carbonated apatite particles having a diameter of approximately 10 μm, Cytrans® from GC Dental
- calcium carbonate particles having a primary particle size of 200 nm, from Shirai,
- ZnO-containing reactive glass powder mixture, comprising glass from Schott (85 wt-% of the mixture) and GC Dental (15 wt-% of the mixture)
- silica filler: $BaAlSiO_2$ filler particles (diameter 0.7 μm) from Schott (UltraFine GM27884, Schott, Landshut, Germany)
- various resin mixtures comprising
    - BisGMA (bisphenol A-glycidyl methacrylate) from Esstech Inc. (Essington, PA, USA)
    - TEGDMA (diethylene glycol dimethacrylate, triethylene glycol dimethacrylate) from Esstech Inc. (Essington, PA, USA)
    - UDMA (urethane dimethacrylate) from Sigma-Aldrich Co. (St Louis, MO, USA)

The dental discs used in the testing were prepared as follows. Firstly, the occlusal surfaces of extracted teeth were wet ground using an automatic grinding machine, 500-grit, 300 rpm under water cooling (Struers Rotopol-11) from extracted sound wisdom teeth (acquired from the teaching clinic of Institute of Dentistry, University of Turku, Finland). Thereafter, discs (having a thickness of 2 mm) were cut from the teeth in cross direction of the longest dimension of a tooth, 5 parallel samples for each experiment, as shown in Figure 2A, which illustrates a tooth 3 and two cutting lines 4. Demineralisation of the dentin discs was simulated by acid etching (37 % phosphoric acid for 20 seconds).

For remineralisation testing, the discs (as shown in Figure 2B with reference number 5) were either surface treated (i.e. rinsed) with an activation agent solution (see Table 3) or without (i.e. rinsed with plain water; as control samples C0) before immersion in simulated body fluid (SBF) prepared according to ISO 23317 (2014) for seven days.

For assessing the interface between the dentin and the present lining material, interface samples were prepared by first treating dentin discs (prepared as above) with PAA (450000 g/mol, 10 mg/l in water), then with a lining material according to Exp 30 (see Table 1 below). As the lining material was flowable, it was applied on the dentin discs by using a hand-held dental plastic instrument, followed by light-curing for 20 seconds (emission wavelength range was 430-480 nm, irradiance approximately 1600 $mW/cm^2$). Thereafter, the samples were immersed in SBF for two weeks.

Comparative interface samples were also prepared for observing the interface between a given commercial material and the dentin. The dentin discs were prepared as explained above, using phosphoric acid etching, but no activation agent solution. The commercial materials were applied on the dentin discs according to the manufacturer's instructions, and the cured/hardened (depending on the material) comparative samples were stored two weeks in SBF. Comparative example C1 used Fuji II LC from GC Dental, comparative example C2 used Fuji IX from GC Dental, comparative example C3 used Activa-Liner from Pulpdent Corp. US and comparative example C4 used Caredyne from GC Dental.

Ion releasing (or dissolution) from the present materials and a control sample was tested by preparing samples according to ISO 4049:2019, having a diameter of 10 mm and a thickness of 1 mm. The material was arranged in a metal mould and cured for 20 seconds.

The ion releasing samples according to the present description had different component contents, as listed in Table 1. The amounts of components of the lining material are in weight-percentages of the total weight of the lining material. The resin used was BisGMA/TEGDMA 50/50 for Exp 24, 25, 26, 27, 28 and 30 and UDMA/TEGDMA (70/30) for Exp 31. For testing dissolution of calcium to water in 24 hours from disc samples, a control ion releasing sample C5 was used, which consisted of BisGMA/TEGDMA (50/50) and silica filler.

Table 1

| | Exp 24 | Exp 25 | Exp 26 | Exp 27 | Exp 28 | Exp 30 | Exp 31 | C5 |
|---|---|---|---|---|---|---|---|---|
| E-glass fibres (wt-%) | 20 | 20 | 20 | 20 | 20 | 20 | 20 | |
| Carbonated apatite (wt-%) | 20 | 20 | 20 | | 10 | 18 | 18 | |
| Calcium carbonate (wt-%) | | | | 20 | 10 | 2 | 2 | |
| ZnO-containing glass powder (wt-%) | | 5 | 20 | 20 | 20 | 20 | 20 | |
| Silica filler | 20 | 15 | | | | | | 60 |

(continued)

| | Exp 24 | Exp 25 | Exp 26 | Exp 27 | Exp 28 | Exp 30 | Exp 31 | C5 |
|---|---|---|---|---|---|---|---|---|
| Resin (wt-%) | 40 | 40 | 40 | 40 | 40 | 40 | 40 | 40 |

For testing flexural strength, samples identical to the above samples Exp 24, 25, 26, 27, 28 and 30 were used (i.e. prepared according to ISO 4049:2019). Further Comparative flexural strength samples (each 5 parallel samples) were also used. The Comparative flexural strength samples were prepared using Fuji lining LC from GC Dental as C6, TheraCal LC from Bisco Dental, US as C7, Activa-Liner from Pulpdent Corp., US as C8 and Ultra-Blend plus from Ultra Dent, US as C9. Furthermore, Comparative flexural strength samples were prepared using the same materials as in Comparative samples C1-C4, namely Comparative example C10 used Fuji II LC from GC Dental, comparative example C11 used Fuji IX from GC Dental, and comparative example C12 used Caredyne from GC Dental.

The bonding performance (i.e. shear-bond strength) between the material according to the present description and the dentin was also evaluated after different dentin surface treatments. The bonding performance was also assessed by adding 10-MDP (10-methacryloyloxydecyl dihydrogen phosphate) with different weight percentages to the PAA solution.

Firstly, dentin discs were prepare as explained above, with the exception that etching was only used for one bonding sample and one Comparative bonding sample (as listed below in Table 2, samples S2a and S2b).

For each treatment, a bonding sample was prepared by applying a lining material according to Exp 30 above (samples S1a-S8a) and a Comparative bonding sample was prepared by using commercial flowable composite (G-aenial Injectable from GC) (samples S1b-S8b) as explained below. Each sample and comparative sample was stored in water overnight, and the bonding strength was measured the following day.

For the bonding performance measurement, the occlusal surfaces of extracted teeth were wet ground (using an automatic grinding machine, 500-grit, 300 rpm under water cooling; Struers Rotopol-11) to create a flat dentin surface. Subsequently, the teeth were mounted individually into an acrylic block (diameter 2.5 cm) using cold cure auto-polymerised acrylic resin from Palapress; Heraus Kulzer, Wehrheim, Germany

By using a transparent polyethylene mould with an inner diameter of 3.6 mm, increments of two mm of lining material (according to Exp 30 above) or comparative material (G-aenial Injectable from GC Dental) were applied to the dentin substrates, to form a protrusion having a diameter of 3.6 mm. The lining materials and comparative materials were photopolymerised twice at full thickness from the side and top surfaces for 20 s using a hand- light-curing unit (Elipar S10). The specimens were stored in water (37°C) for one day prior to testing.

The bonding performance tests were carried out on samples prepared using different dentin surface treatments, according to Table 2.

Table 2

| Dentin surface treatment | Experiment with Exp 30-lining material | Comparative experiment with G-aenial Injectable |
|---|---|---|
| Only PAA (450000 g/mol, 10 mg/l) | S1a | S1b |
| etching + PAA (450000 g/mol, 10 mg/l) | S2a | S2b |
| 0.4 mg/ml MDP + PAA (450000 g/mol, 10 mg/l) | S3a | S3b |
| 2.5 mg/ml MDP + PAA (450000 g/mol, 10 mg/l) | S4a | S4b |
| 5 mg/ml MDP + PAA (450000 g/mol, 10 mg/l) | S5a | S5b |
| 8.3 mg/ml MDP + PAA (450000 g/mol, 10 mg/l) | S6a | S6b |
| 5 mg/ml MDP | C13a | C13b |
| 5 mg/ml MDP + PAA (4000000 g/mol, 10 mg/l) | S7a | S7b |

(continued)

| Dentin surface treatment | Experiment with Exp 30-lining material | Comparative experiment with G-aenial Injectable |
|---|---|---|
| 5 mg/ml MDP + PAA (450000 g/mol, 20 mg/l) | S8a | S8b |
| 5 mg/ml MDP + PAA (450000 g/mol, 2 g/l) | S9a | S9b |

Fracture toughness was also tested using single-edge-notched-beam specimens (2.5 x 5 x 25 $mm^3$) according to an adapted ISO 20795-2 standard method (ASTM 2005). A custom-made stainless steel split mould was used, which enabled the specimen's removal without force. An accurately designed slot was fabricated centrally in the mould extending until its mid-height, which enabled the central location of the notch and optimisation of the crack length (x) to be half of the specimen's height. The tested materials were as in Exp 30 and the comparative tests C1, C2, C3 and C4 above. The material was inserted into the mould placed over a Mylar-strip-covered glass slide in one increment. Before polymerisation or setting, a sharp and centrally located crack was produced by inserting a straight edged steel blade into the prefabricated slot of the mould. Polymerisation of the Exp 30 resin composite was carried out for 20 s in five separate overlapping portions. The upper side of the mould was covered with a Mylar strip and a glass slide from both sides of the blade, before being exposed to the polymerisation light. Upon the removal from the mould, each specimen was polymerised also on the opposite side. Application and polymerisation of commercial materials (C1-C4) was made according to manufacturer's instructions. The specimens from each material (n=6) were stored dry at 37°C for 24 h before testing.

One further sample, C14, was prepared for testing effect of cellulose nanocrystals (NCC) on reduction of aerosol release during use of the activation agent solution. In this example, the activation agent solution was prepared by mixing distilled water (grade III) and polyacrylic acid (PAA) from Sigma-Aldrich (St. Louis, MO, USA) having a molecular weight of 450000 g/mol to a concentration of 10 mg/l of the PAA in water. Additionally, 10-methacryloyloxydecyl dihydrogen phosphate (MDP) from Fluorochem Ltd, Hadfield, England was added to activation solution, in an amount of 5 mg/ml. The activation solution was thickened with cellulose nanocrystals (NCC), NovaWire-CNC-T from Novarials in an amount of 1 wt-% of the total weight of the solution. This sample was further used for shear-bond testing as described below, using light-cured G-aenial Injectable from GC, diameter of the stub 3.6 mm. The sample C14 is thus comparable to the sample C13b, with the exception of use of the NCC.

**Test methods**

The chemical properties were estimated by measuring a degree of monomer conversion (DC %) by FT-IR spectrometry using the method described in Ferracane et al., Academy of Dental Materials guidance-Resin composites: Part II-Technique sensitivity (handling, polymerization, dimensional changes), Dental Materials 33 (2017) 1171-1191.

Further, ion-releasing with pH-change, using ion-selective electrode (ISE) and atomic absorption spectroscopy (AAS), using ISO 9917-1:2007 were measured.

Scanning electron microscope (SEM/EDS) were used for characterisation of the dentin surface (both the mineralisation control samples C0 and the mineralisation samples rinsed with the activation agent solution) after 1, 2, 3 and 7 days of immersion in the SBF.

The interface between the material according to the present description and the dentin was tested after storage either for two weeks, of interface samples in SBF, by breaking the disc into two halves horizontally and using SEM/EDS and chemical mapping. The same was done for the comparative interface samples C1-C5.

For the measurement of dissolution of calcium and zinc from the samples, the following tools and reagents were used. AAS (atomic absorption spectroscopy) values were measured using AAnalyst 400 Atomic Absorbtion Spectrometer, Serial no. 201S8090503, PerkinElmer Life and Analytical Sciences, Shelton, CT, USA. ISE (ion-selective electrode) values were measured using Orion ionplus Sure-Flow Electrode Body 9700BNWP, VV1-11811, ThermoScientific, USA, while the liquid for the electrode was Orion ionplus Filling Solution, A optimum Results, Orion 900061, 60 ml, LOT UV1, P/N 223228-A01, Thermo Fisher Scientific, Chelmsford, USA. The calcium standard used was Calcium Standard 0.1M Ca2+, Orion ionplus Application Solution, 475 ml, Orion 922006, (Deionized Water H2O CAS 7732-18-5, Calcium Chloride CaC12 CAS 10043-52-4), Exp 11/2018, LOT: UP1, P/N: 02480-A03, CML: 922006, Thermo Fisher Scientific, USA. For ISA, also Calcium ISA Ionic Strength Adjuster, Orion ionplus Application Solution, 475 ml, Orion 932011, LOT: UP1, P/N: 702555-A03, CML: 932011, Thermo Fisher Scientific, USA was used. The pH-meter used was PHM 220 LAB pH METER, 657R005N035, Radiometer Copenhagen, MeterLab TM, Radiometer Analytical S.A. France,

with a buffer solution at pH 4 AVS (Titrinorm, VWR Chemicals, 00168, 100 ml, 32095.184, LOT 19B194007, VWR International S.A.S. Fontenay-sous-Bois France). Another buffer solution (phosphate buffer) at pH 7 was also used (+/- 0,02(20C): AVS Titrinorm, VWR Chemicals, 00152, 100 ml, 32096.187, LOT 19G124120, VWR International bvba Leuven Belgium) as well as a buffer solution pH 10 (AVS Titrinorm, VWR Chemicals, 00093, 100 ml, 32040.185, LOT 19G104115, VWR International bvba Leuven Belgium).

The mechanical properties were estimated using measurement of flexural strength before and after water ageing at 100 °C for 16 hours, using a model LRX, Lloyd Instruments Ltd and ISO standard 4049.

The measurement of bonding performance (shear-bond strength) was carried out as follows. The samples and comparative samples prepared as explained above were first mounted and secured in a mounting jig and then placed on the shear-bond strength test assembly. The test was performed using a universal testing machine (Model LRX, Lloyd Instruments) at room temperature (23 ± 1 °C), and the data was recorded using PC software (Nexygen, Lloyd Instruments). The shearing rod was placed against and parallel to the flat prepared bonding sites. A circular perforation of diameter 4.1 mm was made into the metal blade through which the composites passed through until the metal blade was positioned at the lining material or commercial material-dentin interface, and then using a span length of 10 mm and crosshead speed of 1.0 mm/min, the specimens were loaded until fracture. Bonding strength was calculated by dividing the maximum load at failure (N) with the bonding area ($mm^2$) and recorded in megapascals (MPa). The test setting is illustrated in Figures 27A and 27B.

An additional test was also carried out, where dentin surface discs were prepared without etching of the surface, i.e. simply bur-cut with high-speed hand piece, under either plain water coolant solution or under the above-described activation agent solution (molecular weight of PAA 450000 g/mol and concentration 10 mg/1). The discs were stored in SBF for one week, and thereafter evaluated with SEM/EDS as described above. The results are shown in Figures 15- 19.

Fracture toughness was also tested on samples prepared as explained above, in three-point bending mode, in a universal material testing machine at a crosshead speed of 1.0 mm/min.

The fracture toughness (FT) was calculated using the Equation:

$$K_{max} - \left[\frac{P \cdot L}{B} \cdot W^{3/2}\right] \cdot f(x)$$

where:

*f* is a geometrical function dependent on *x*:

$$f(x) = 3x^{1/2}\left[1,99 - x(1-x)(2,15 - 3,93x + 2,7x^2\right] / \left[2(1+2x)(1-x)^{3/2}\right]$$

Here P is the maximum load in kilonewtons (kN), L is the span length (2 cm), B is the specimen thickness in centimetres (cm), W is the specimen width (depth) in cm, x is a geometrical function dependent on a/W and a is the crack length in cm. The value of x is given in ASTM E399-12.

## Results

The degree of monomer conversion (DC %) measured by FT-IR spectrometry was 60-61 % for all samples according to Exp. 24, 25, 26, 27, 28 and 30.

Several different concentrations of PAA at different molecular weights were tested for mineralisation (immersion in SBF for seven days). The results are summarised in Table 3, where (-) means no mineralisation, (+) means some mineralisation but not full mineralisation and (++) means that the surface was fully covered with mineralisation (as shown in Figure 5B).

| Concentration \ Molecular weight | 5000 | 450000 | 4000000 |
|---|---|---|---|
| 10 mg/l | - | ++ | - |
| 20 mg/l | - | + | - |
| 2 g/l | - | - | - |
| 500 g/l | - | - | - |

Table 3

Figures 3A to 5B show the remineralisation results after immersion of demineralised dentin disc samples in SBF at various points of time. Figures A are from control samples C0, i.e. samples that were not treated with activation agent solution but only with water. Figures B are from samples treated with the above activation agent solution at 450000 g/mol and 10 mg/l. Figures 3 are after one day of immersion, Figures 4 after three days of immersion and Figures 5 after seven days of immersion. Remineralisation of the control samples C0 (Figures 3A, 4A and 5A) was very limited, only very little remineralisation can be seen after seven days of immersion. The samples treated with the above activation agent solution (Figures 3B, 4B and 5B) showed significant remineralisation, already after three days.

Figure 6A shows the same dentin disc as Figure 5A, but as a side view. Figure 6B shows the dentin disc of Figure 5B, as a side view. As can be seen, the dentin tubules in Figure 6B have been remineralised at the surface of the disc, while those of Figure 6A essentially remain open.

Figures 7A and 7B (of which Figure 7B is a partial enlargement of Figure 7A as shown) illustrate the interface between the dentin treated with the activation agent solution (lower part of the Figures, in lighter grey) and the lining material according to the present invention (as in Exp 30 after two weeks of storage in SBF) as applied (upper part of the Figures, where filler particles are shown in light grey). As can be seen, mineralised or reactive (ions-rich) layer was formed at the interface between activated dentin and lining material.

Figures 8, 9 and 10 show the major elemental composition of different parts of the interface (as shown in Figures 7A and 7B), measured by energy dispersive X-ray spectrometer (EDX). Figure 8 is measured from area 1 of Figure 7B (in the upper part of the Figure) and shows the composition of the ion releasing material. Figure 9 is measured at point 5 of Figure 7B (in the middle of the Figure), and thus shows the composition of the mineralised layer at the interface. Figure 10 is measured from area 2 of Figure 7B (in the lower part of the Figure) and shows the composition of the demineralised dentin. As can be seen, the major elemental compositions of the ions-rich layer were Ca and P, which promote the remineralisation of activated dentin.

Figures 11-14 show the interface between a commercial material (Comparative examples C1-C4) and the dentin. Figure 11 is uses Fuji II LC from GC Dental (C1), Figure 12 Fuji IX from GC Dental (C2), Figure 13 Activa-Liner from Pulpdent Corp. US (C3) and Figure 14 Caredyne from GC Dental (C4). The results show that no reactive or mineralised layer was formed at the interface between these commercial materials and dentin.

Further results on remineralisation tests are shown in Figures 15 and 16 (at different magnification) show the dentin surface that was cut under plain water cooling and immersed in SBF for one week. No mineralisation is visible, only a smear layer. Figures 17 and 18 are the same with SEM/EDS-photos as Figures 15 and 16, but for a sample that was cut under the present activation agent solution and stored in SBF for one week. Mineralisation can be seen to cover the whole surface of the sample. Figure 19 is a cross-sectional view of the same sample, showing the thickness of the mineralised layer. Figure 20 shows the major elemental composition of the area marked with square in Figure 19, measured by EDX. As can be seen, the activation agent solution provides an appropriate environment for dentin remineralisation,

Figure 21 shows the results of testing dissolution of calcium (g/kg) to water in 24 hours, from disc samples. The tests were carried out for four samples, the first sample on the left was from Exp 26, the second sample from Exp 27, the third sample was the control sample C5 and the fourth sample on the right was from Exp 30. For each sample according to the present description, four measurements were made, and the columns in each group show the results. In each group, the left-most column (the smallest for each group) gives the measurement by AAS from the whole weight of the sample, the second column from the lest the measurement by AAS from the weight of the active part of the sample. Naturally, only the AAS from the whole weight of the sample can be given for the control sample. The third column in each group corresponds to the measurement of ISE from the whole weight of the sample, and the right-most column to the measurement of ISE from the weight of the active part of the sample. The line illustrates the measured pH of the water in which the samples were immersed. The results show that the inclusion of calcium carbonate in mixture (Exp 27 and Exp 30) is significant when fast calcium release is sought.

Figure 22 illustrates the results of measurements of dissolution of zinc (g/kg) from samples according to Exp 30 to water at three different moments of time, namely 15 minutes (on the left), 24 hours (in the middle) and 168 hours (on the right). The line gives the pH of the water in which the samples were immersed. In each group of columns, the left column is the measurement by AAS from the whole weight of the sample, and the right column is the measurement by AAS from the weight of the active part of the sample. The results show that the amount of zinc ions released increases with time, which also changes the pH to be more alkaline.

Figure 23 illustrates the results of measurements of dissolution of calcium (g/kg) from samples according to Exp 30 to water at three different moments of time, namely 15 minutes (on the left), 24 hours (in the middle) and 168 hours (on the right). The line gives the pH of the water in which the samples were immersed. In each group of columns, the left-most column is the measurement by AAS from the whole weight of the sample and the second column is the measurement by AAS from the weight of the active part of the sample. The third column in each group corresponds to the measurement of ISE from the whole weight of the sample, and the right-most column to the measurement of ISE from the weight of the active part of the sample. The results show that the amount of calcium ions released is increasing with time, which also changes the pH to be more alkaline.

Figure 24 shows flexural strength (in MPa) of various comparative examples (C6-C9) and of several samples prepared according to the present description (Exp 24, Exp 25, Exp 26, Exp 27, Exp 28 and Exp 30), having differing components and component contents. For each sample, the flexural strength was measured on a dry sample (left column in each group, upper line for the numerical results) and on a sample aged by boiling in water (100 °C) for 16 hours (right column in each group, lower line for the numerical results). The results show that the present lining materials are much stronger than commercial comparative materials before and after ageing (boiling).

Figure 25 illustrates the flexural strength (in MPa) of two samples prepared according to the present description (Exp 30 and Exp 31), where the resins were different. For each sample, the flexural strength was measured on a dry sample (left column in both groups) and on a sample boiled in water (100 °C) for 16 hours (right column in both groups). The samples were otherwise identical, but the sample which results are shown in the group of columns on the left used a resin mixture of BisGMA/TEGDMA (50/50) (Exp 30) and the sample which results are shown in the group of columns on the right used a resin mixture of UDMA/TEGDMA (70/30) (Exp 31).

Figure 26 illustrates the flexural strengths (in MPa) for the comparative samples C10, C11, C8 and C12, and also shows the result for the sample according to Exp 30. As can be seen, the present material leads to significantly improved flexural strength, when compared to some commercial materials, and reaches the same flexural strength compared to other commercial materials.

Figure 28 shows the shear-bond strength in MPa for samples S1-S6 and C13, i.e. dentin surface after various surface treatments, as listed above in Table 2. In each group of samples, the left column illustrates the results of samples treated as in Exp 30 (S1a, S2a, S3a, S4a, S5a, S6a, C13a) while the right column illustrates the results of samples treated with the commercial flowable composite (S1b, S2b, S3b, S4b, S5b, S6b, C13b). The results show that when only PAA was used for the dentin surface treatment, both materials lead to the same shear-bond strength (samples S1). When etching was used, the present material leads to significantly improved shear-bond strength (samples S2). The same effect is true for when MDP is used in either 0.4 mg/ml (samples S3), 2.5 mg/ml (samples S4) or 5.0 mg/ml (samples S5), with PAA. For MDP used in an amount of 8.3 mg/ml (samples S6) with PAA or in an amount of 5 mg/ml alone (samples C13), the present materials lead to slightly lower shear-bond strength than with the commercial product.

Figure 29 illustrates also the shear-bond strength in MPa as in Figure 28, for different samples (except for samples S5). This Figure thus illustrates the effect of the concentration of the PAA and its molecular weight. It can be seen that PAA in an amount of 2 g/l gives the lowest results (samples S9), while at concentration of 10 mg/l, the molecular weight of 450000 g/mol (samples S5) gives better shear-bond strength than molecular weight of 4000000 g/mol (samples S7). Similarly, at molecular weight of 450000 g/mol, the shear-bond strength is higher for a concentration of 10 mg/l (samples S5) than for 20 mg/l (samples S8).

Additionally, it can be said that for sample C14 (where NCC was used in the activation agent solution), the shear-bond value was 3.9 MPa (SD 2.2). Thus, it gives the same result as for sample C13b, which differs from C14 only in the use of NCC. Hence, the use of NCC did not have any effect on the shear-bond strength of the material.

Figures 30A, 30B, 31A and 31B are SEM photos of dentin surfaces, after treatment with either 5 mg/ml of MDP (i.e. C13, Figures 30A and 30B at different magnifications) or after treatment with 5 mg/ml of MDP and PAA (i.e. S5; Figures 31A and 31B at different magnifications). Both samples were immersed in SBF for one week. As can be seen, the samples treated with both MDP and PAA show significantly more mineralisation than when MDP was used alone.

Figure 32 illustrated the fracture toughness of the comparative samples C10, C11, C8 and C12, as well as of the sample prepared using the lining material of Exp 30, given in MPa $m^{1/2}$ As can be seen, the fracture toughness of the material according to the present description is significantly higher than for the commercial materials.

## Claims

1. A kit for dental restoration comprising

   - an aqueous activation agent solution comprising polyacrylic acid having a molecular weight of 100000-1000000 g/mol, wherein the concentration of the polyacrylic acid in the solution is 1-30 mg/l, and
   - a lining material comprising 20-80 wt-% of a dental resin and 20-80 wt-% of an ion releasing material, which ion releasing material comprises at least one of calcium, phosphate and zinc, based on the total weight of the lining material.

2. The kit according to claim 1, wherein the molecular weight of polyacrylic acid is 250000-600000 g/mol.

3. The kit according to claim 1 or 2, wherein concentration of the polyacrylic acid in the solution is 5-20 mg/l.

4. The kit according to any of the preceding claims, wherein the activation agent solution further comprises 10-methacryloyloxydecyl dihydrogen phosphate in a concentration of 0.8-7.5 mg/ml.

5. The kit according to any of the preceding claims, wherein the ion releasing material is selected from hydroxyapatite, carbonated apatite, calcium carbonate, bioactive glass, zinc oxide containing glass, aluminium calcium silicate glass, titanium oxide containing glass and mixtures thereof.

6. The kit according to any of the preceding claims, wherein the composition of the ion releasing material is

   - 35-55 wt-% of carbonated apatite,
   - 2-10 wt-% of calcium carbonate, and
   - 40-60 wt-% of ZnO containing glass,

   based on the total weight of the ion releasing material.

7. The kit according to any of the preceding claims, wherein the dental resin is selected from a group consisting of methyl methacrylate, ethyl methacrylate, n-butyl methacrylate, isobutyl methacrylate, 2-ethylhexyl methacrylate, cyclohexyl methacrylate, isobornyl methacrylate, tetrahydrofurfuryl methacrylate, benzyl methacrylate, morpholinoethyl methacrylate, diethylene glycol dimethacrylate, triethylene glycol dimethacrylate, diurethane dimethacrylate, 2,2-bis(4-(2-hydroxy-3-methacryloxy)phenyl)propane, acrylic acid, epoxy, bisphenol A-glycidyl methacrylate, urethane dimethacrylate, trimethylolpropane ethoxylate triacrylate, semi-crystalline polyceram and mixtures thereof.

8. The kit according to any of the preceding claims, further comprising inert fillers, arranged in the lining material.

9. The kit according to claim 8, wherein the inert fillers are selected from short E- glass fibres, S-glass fibres and mixtures thereof.

10. The kit according to claim 8 or 9, wherein the relative amounts of the ion releasing material, the dental resin and the inert fillers are

    - 30-50 wt-% of the ion releasing material,
    - 30-50 wt-% of the dental resin, and
    - 10-30 wt-% of the inert fillers,

    based on the total weight of the lining material.

11. The kit according to any of the preceding claims, further comprising a dental restorative material.

12. The kit according to any of the preceding claims, wherein the activation agent solution is suitable for use as a drilling solution or as a surface modifier during dental restoration.

13. The kit according to any of the preceding claims, wherein the lining material is suitable for use as a liner material or a base material during dental restoration.

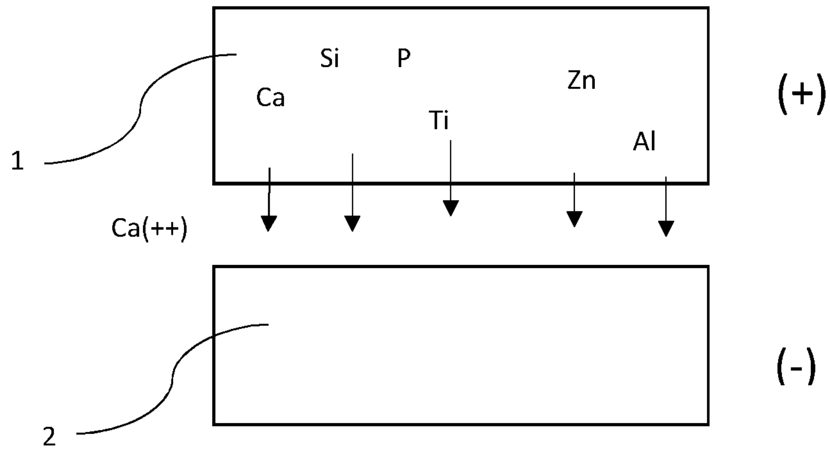


FIG. 1

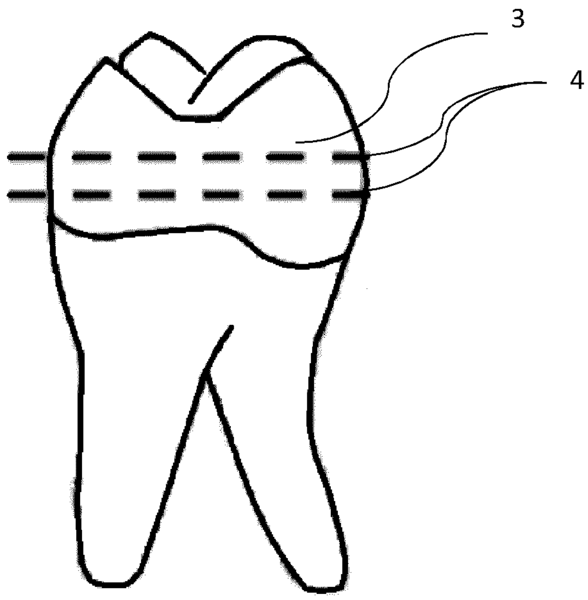


FIG. 2A

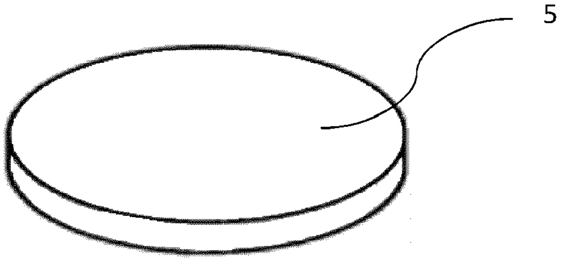


FIG. 2B

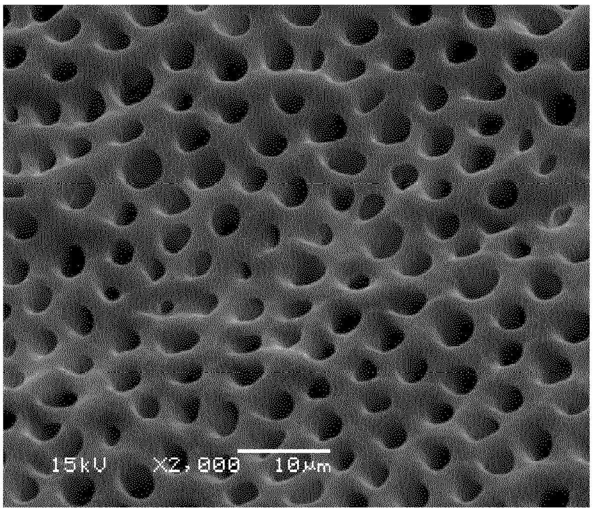


FIG. 3A

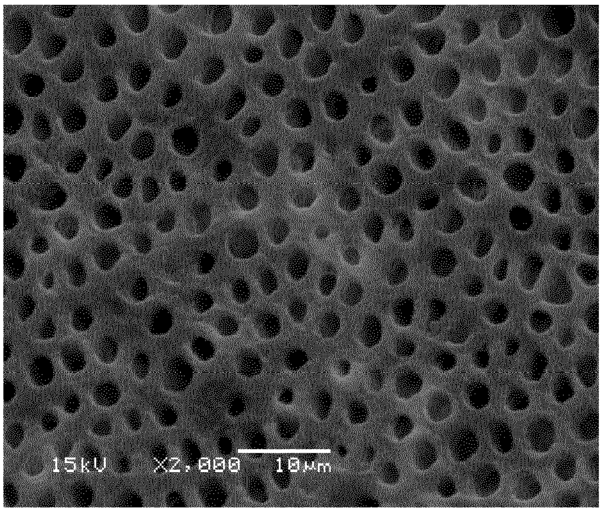


FIG. 3B

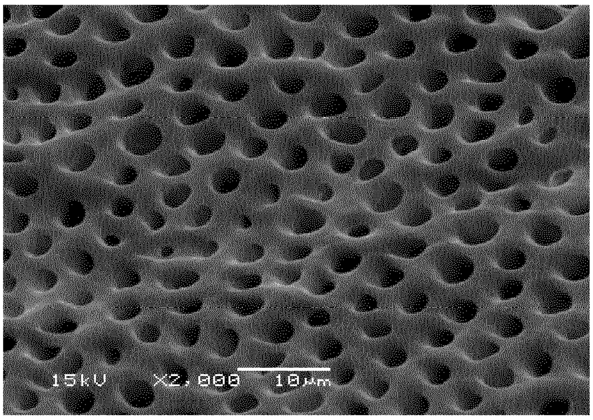


FIG. 4A

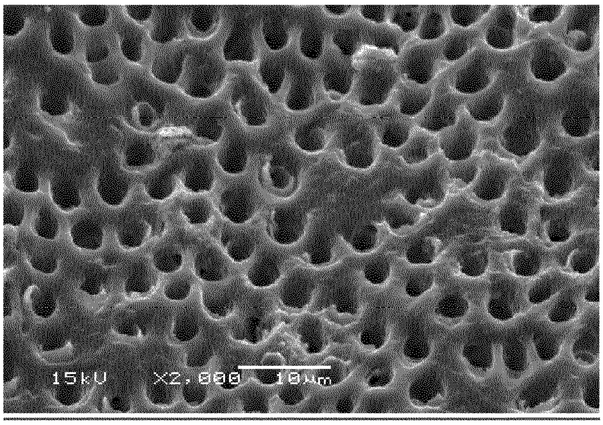


FIG. 4B

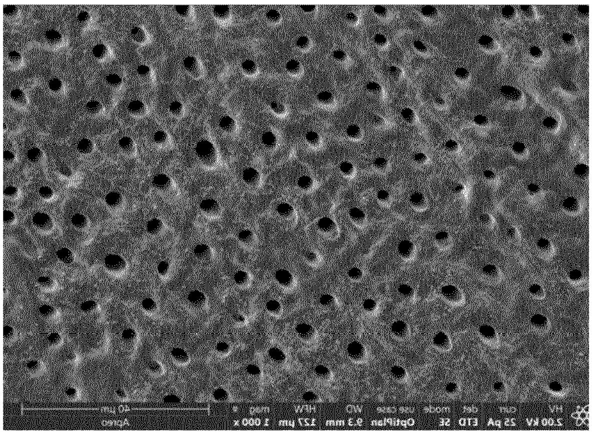

FIG. 5A

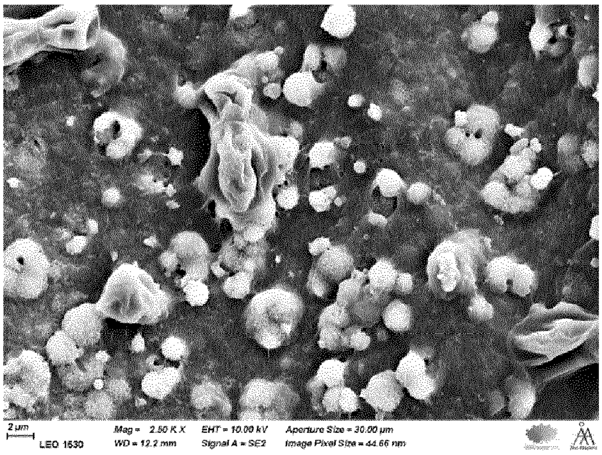

FIG. 5B

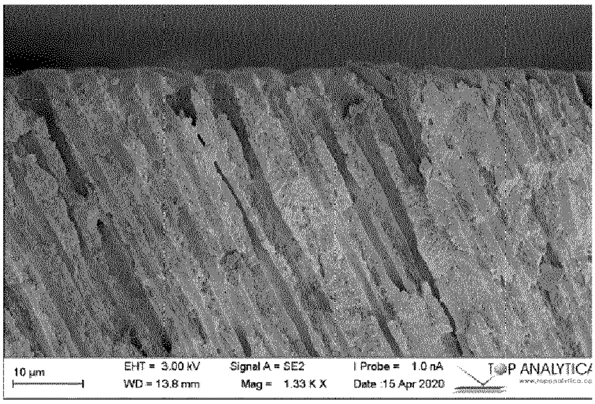


FIG. 6A

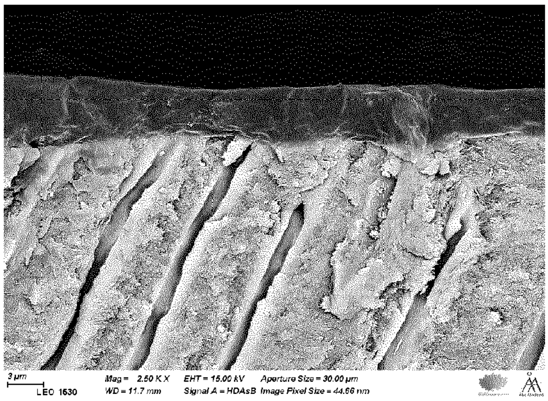


FIG. 6B

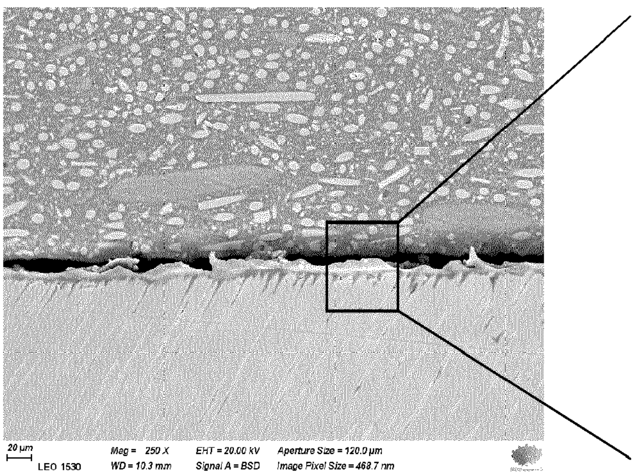


FIG. 7A

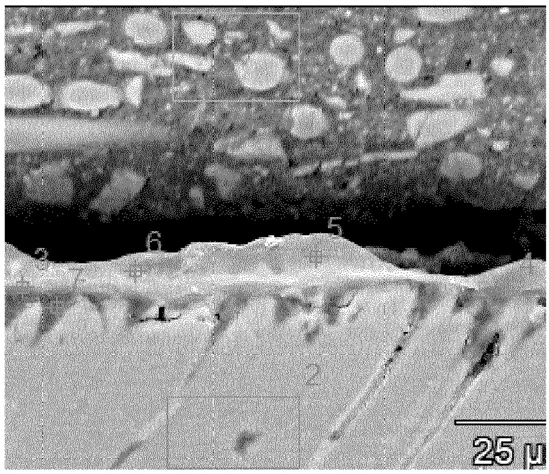


FIG. 7B

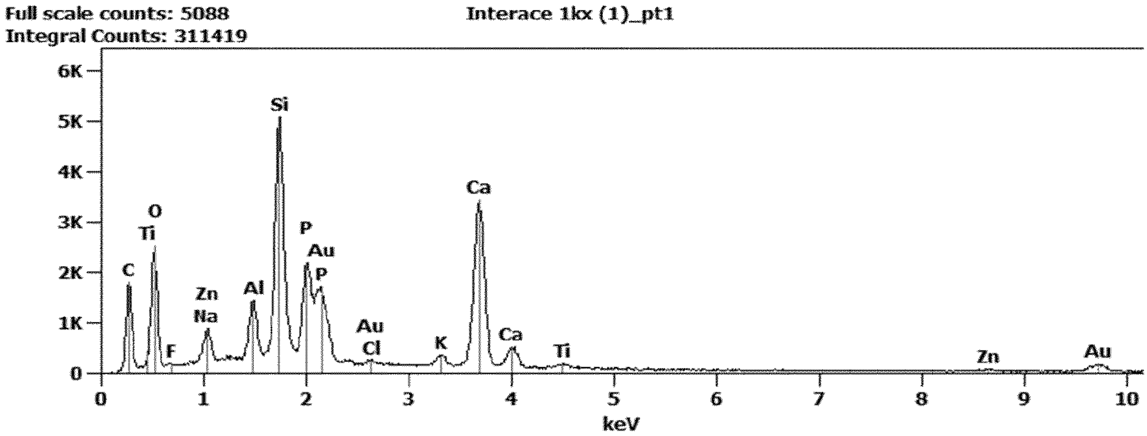


FIG. 8

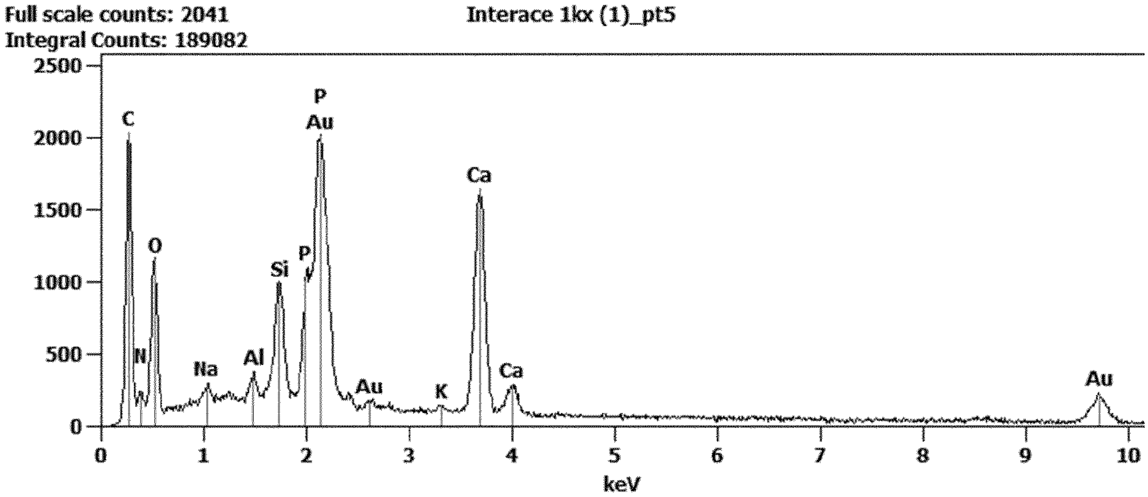


FIG. 9

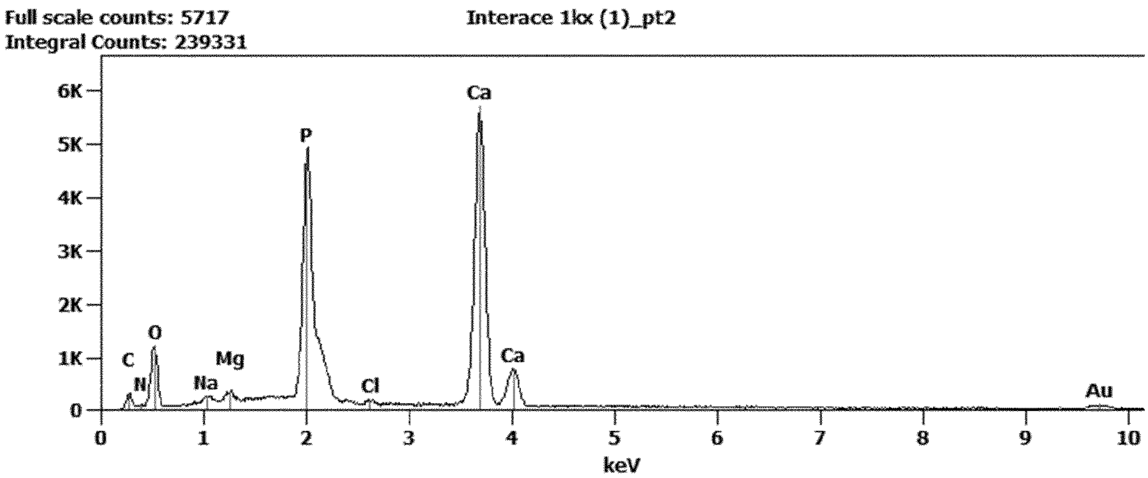


FIG. 10

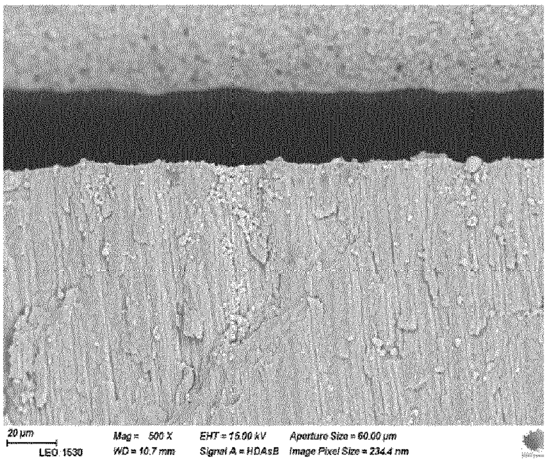


FIG. 11

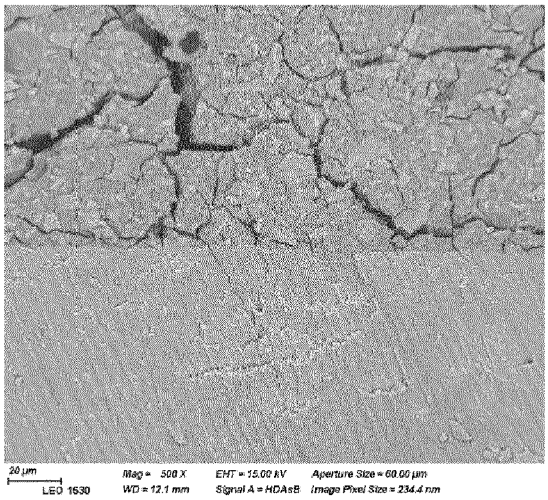


FIG. 12

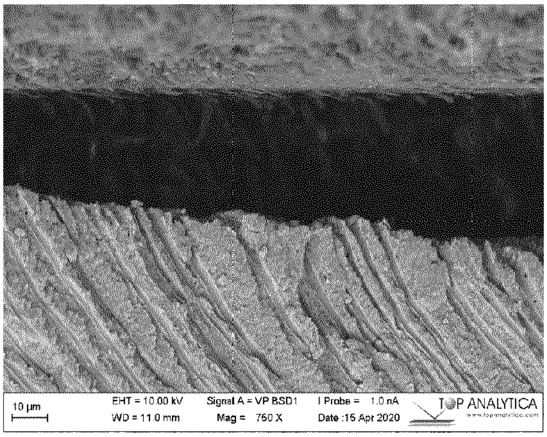


FIG. 13

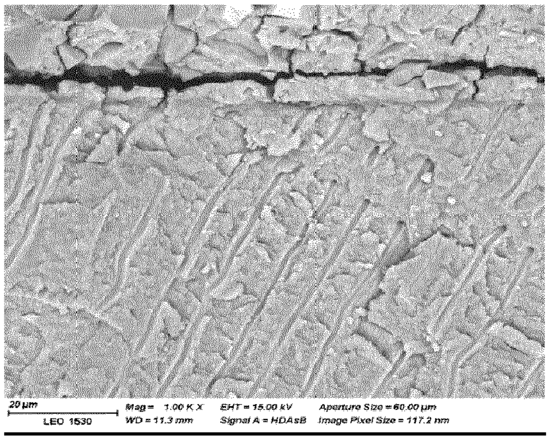


FIG. 14

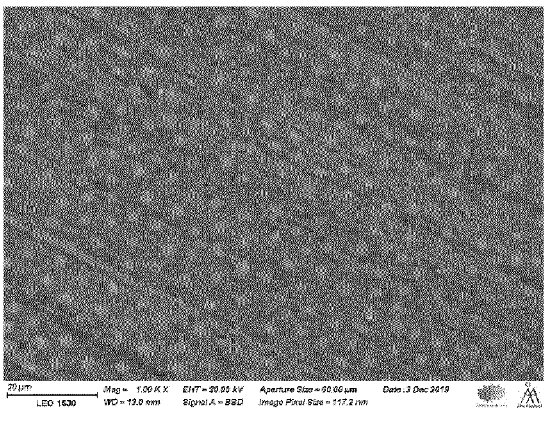


FIG. 15

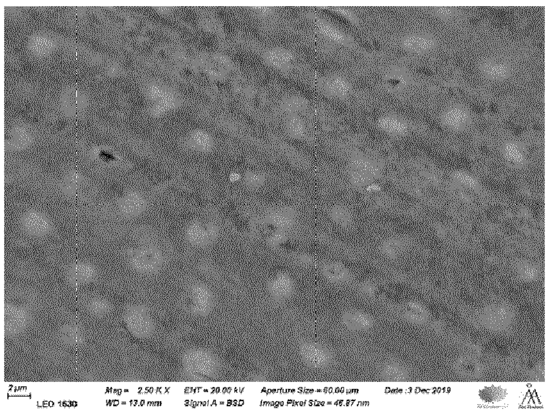


FIG. 16

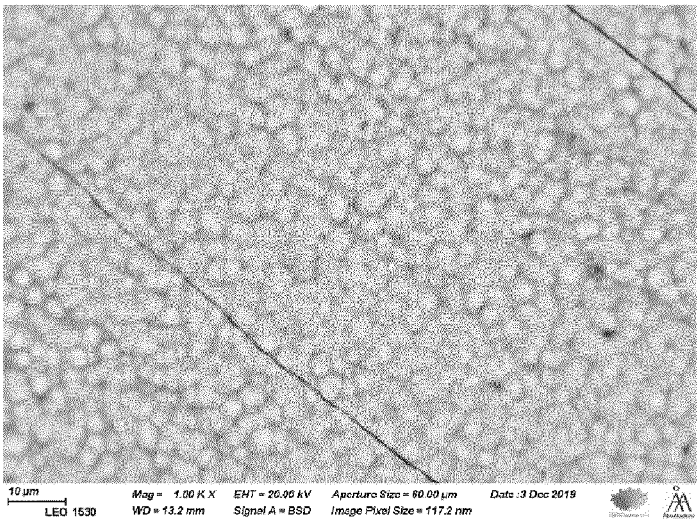


FIG. 17

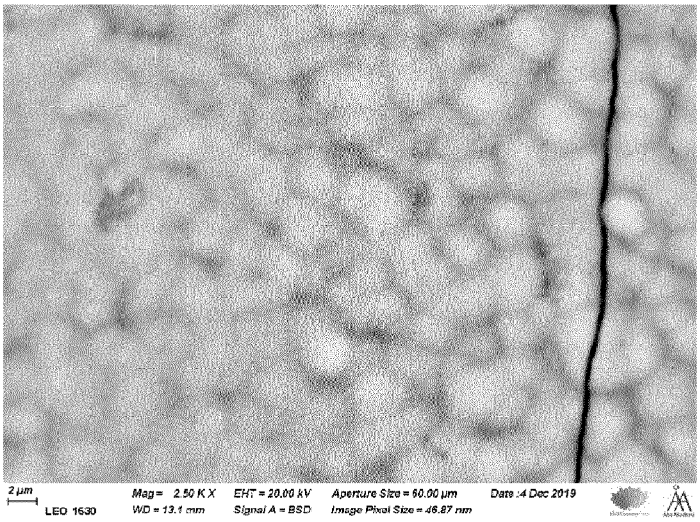


FIG. 18

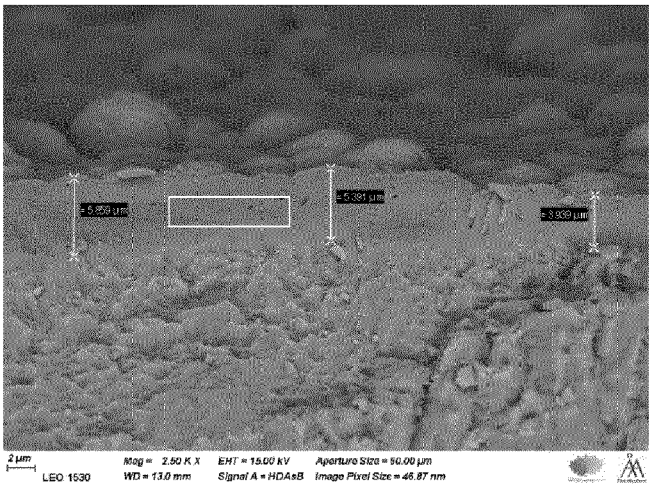


FIG. 19

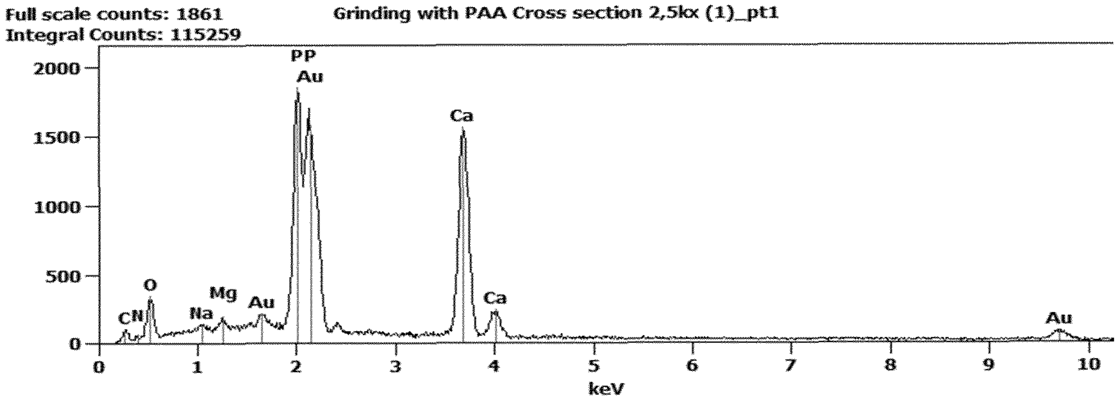
Full scale counts: 1861
Integral Counts: 115259
Grinding with PAA Cross section 2,5kx (1)_pt1
2000
1500
1000
500
0
PP
Au
Ca
O
Mg
Na
Au
CN
Ca
Au
0
1
2
3
4
5
6
7
8
9
10
keV

FIG. 20

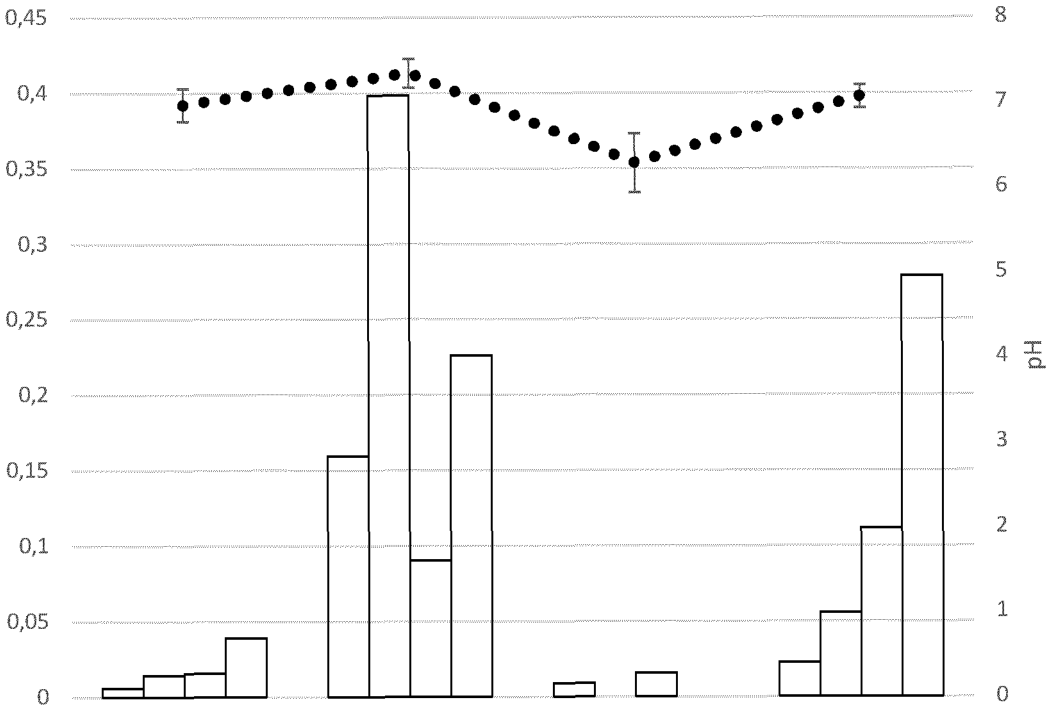
0,45
0,4
0,35
0,3
0,25
0,2
0,15
0,1
0,05
0
8
7
6
5
4
3
2
1
0
pH

FIG. 21

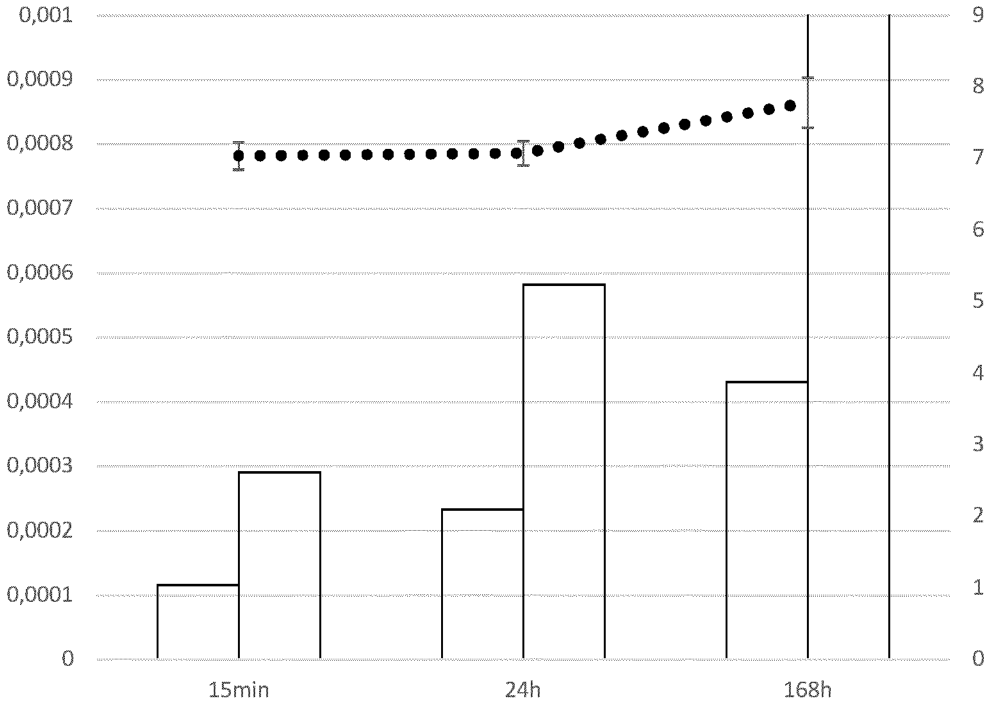
0,001
0,0009
0,0008
0,0007
0,0006
0,0005
0,0004
0,0003
0,0002
0,0001
0
9
8
7
6
5
4
3
2
1
0
15min
24h
168h

FIG. 22

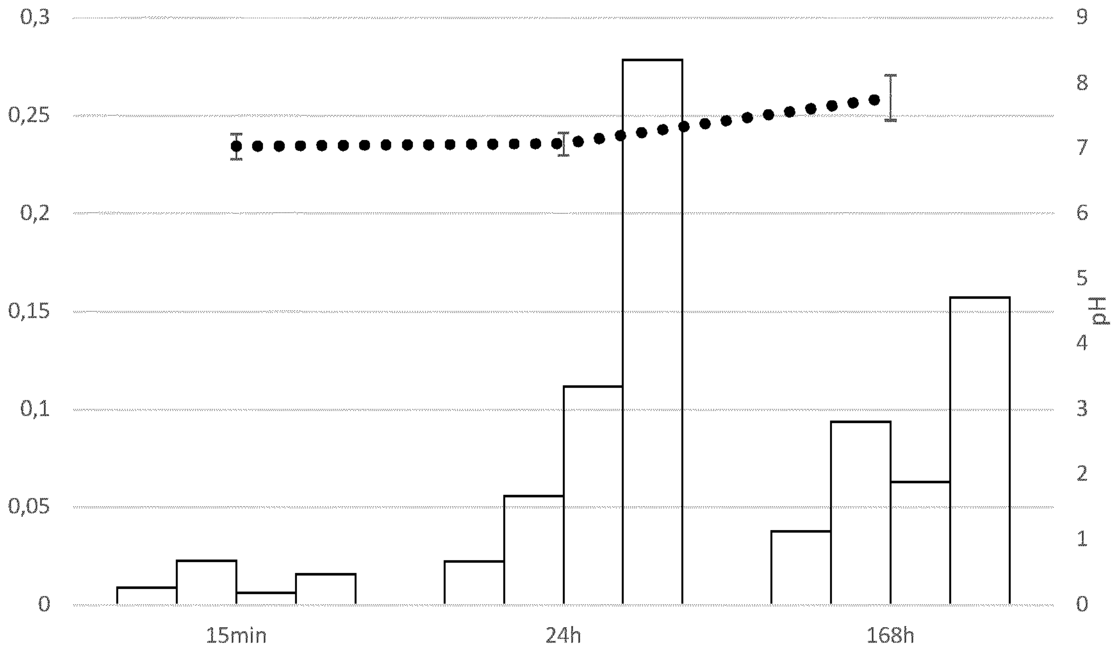
0,3
0,25
0,2
0,15
0,1
0,05
0
9
8
7
6
5
4
3
2
1
0
pH
15min
24h
168h

FIG. 23

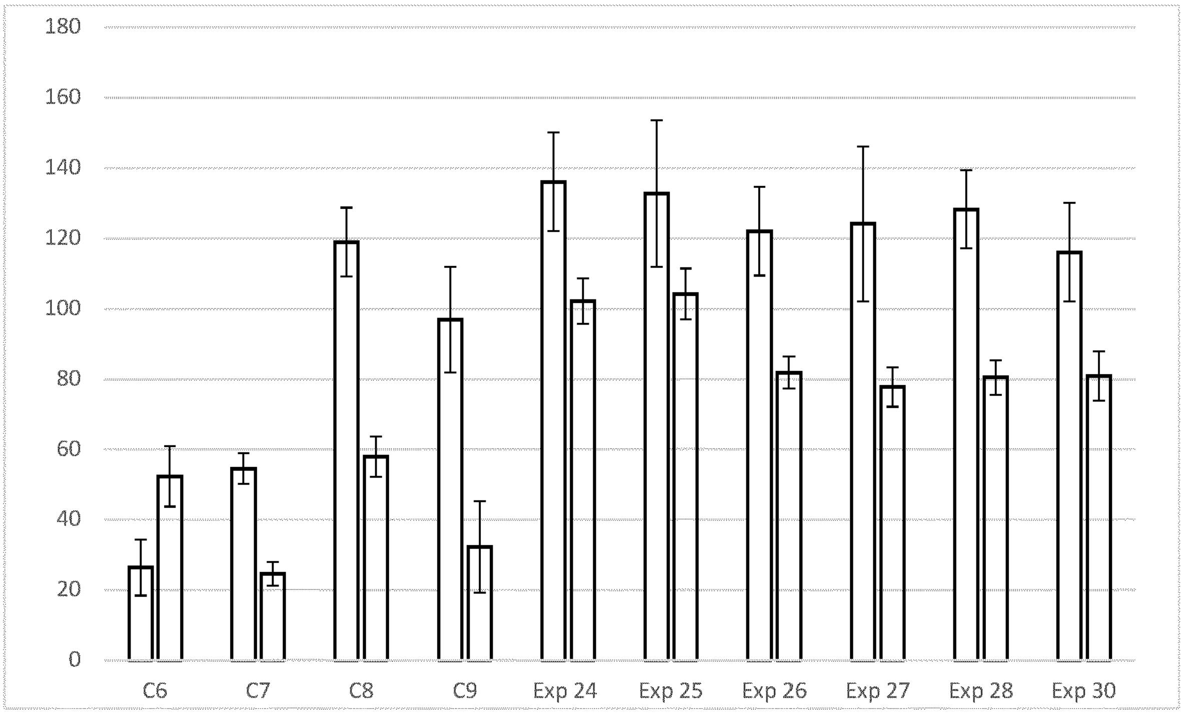
180
160
140
120
100
80
60
40
20
0
C6
C7
C8
C9
Exp 24
Exp 25
Exp 26
Exp 27
Exp 28
Exp 30

FIG. 24

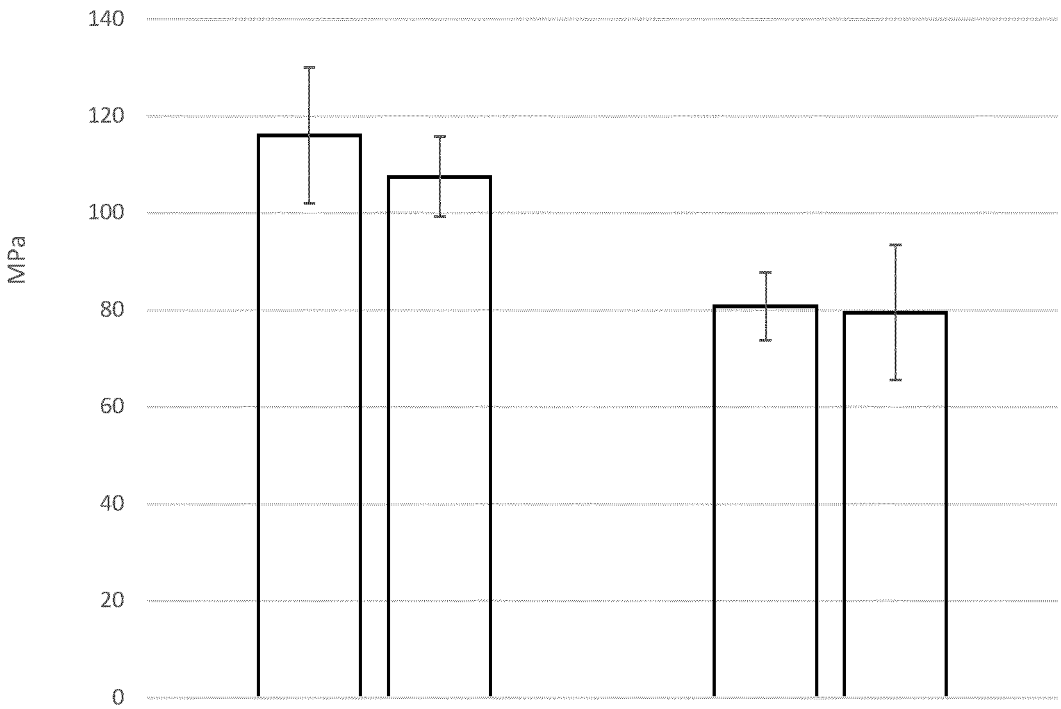
MPa
140
120
100
80
60
40
20
0

FIG. 25

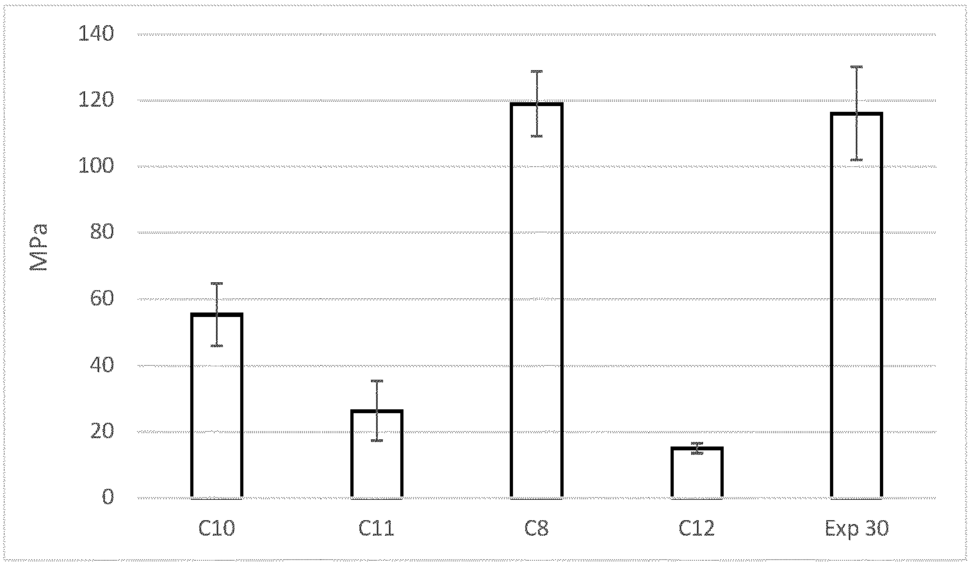


FIG. 26

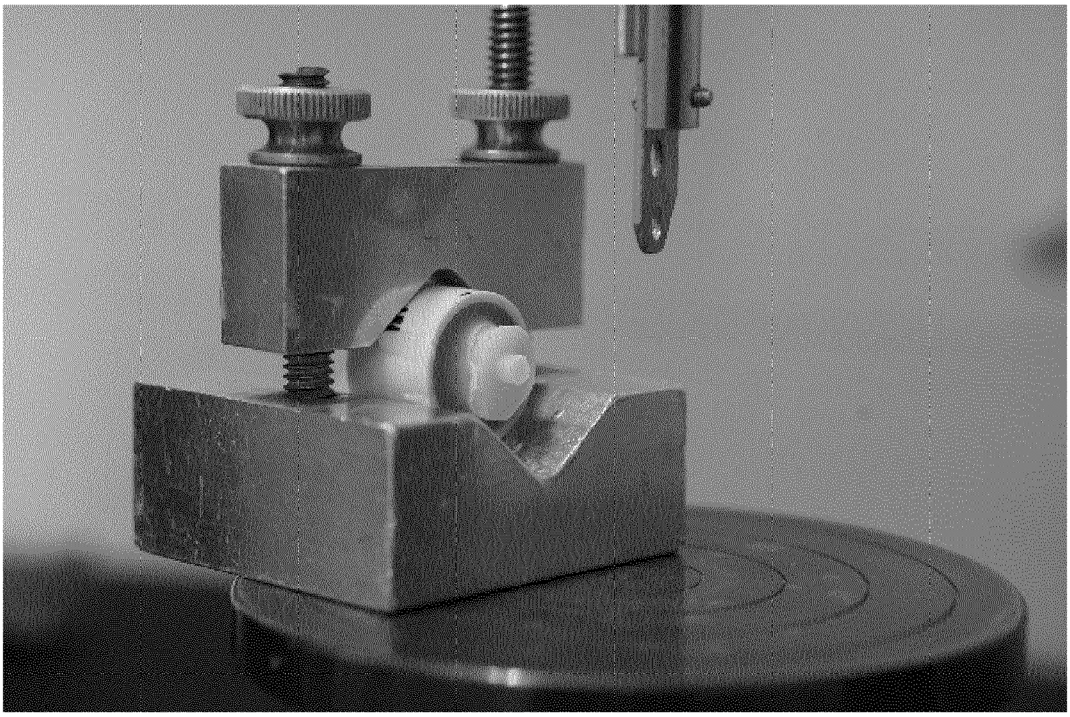

FIG. 27A

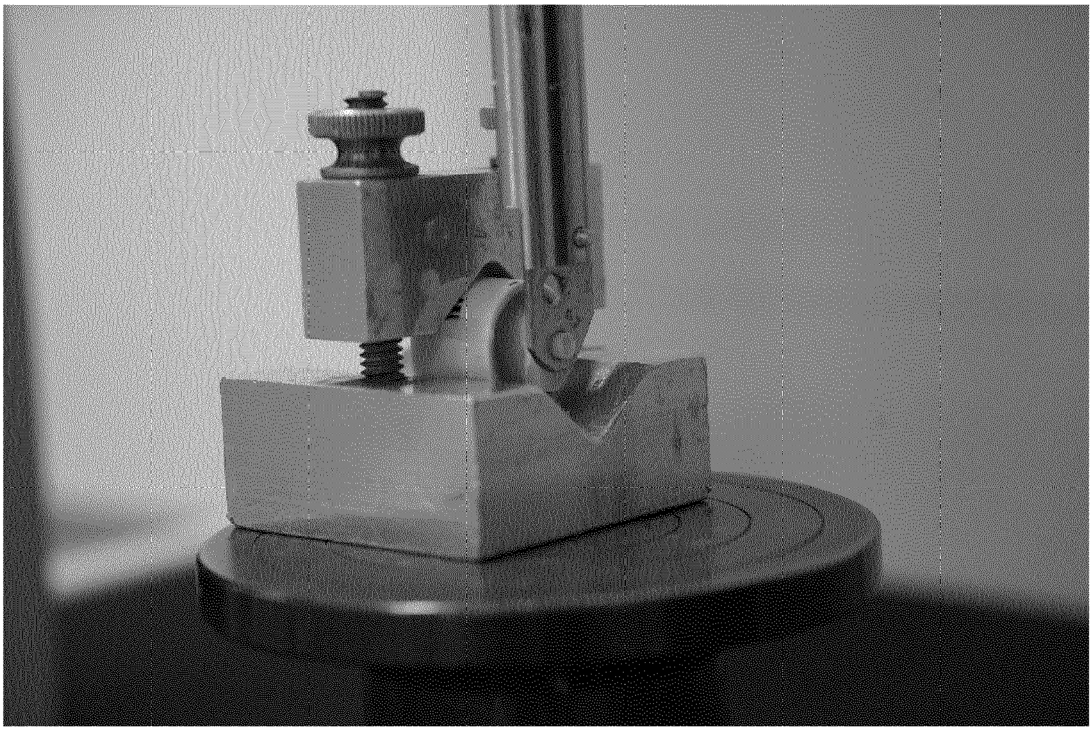

FIG. 27B

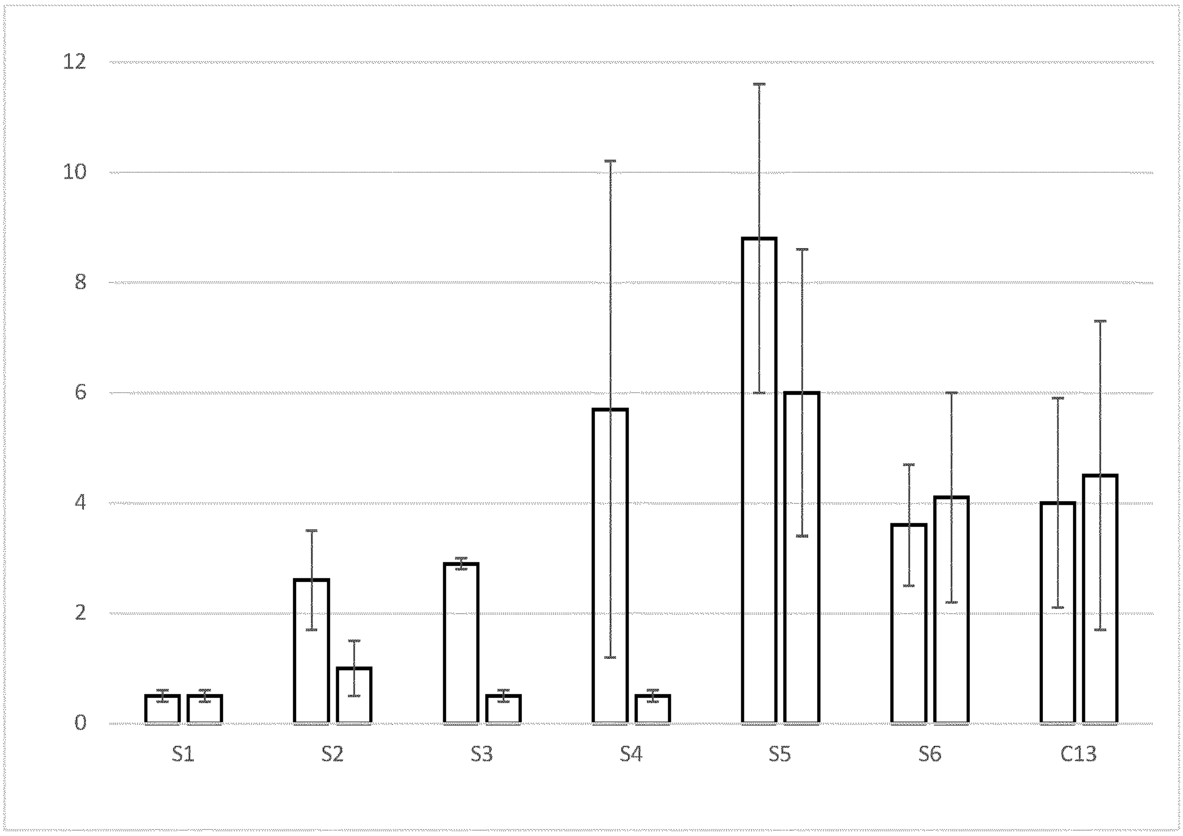


FIG. 28

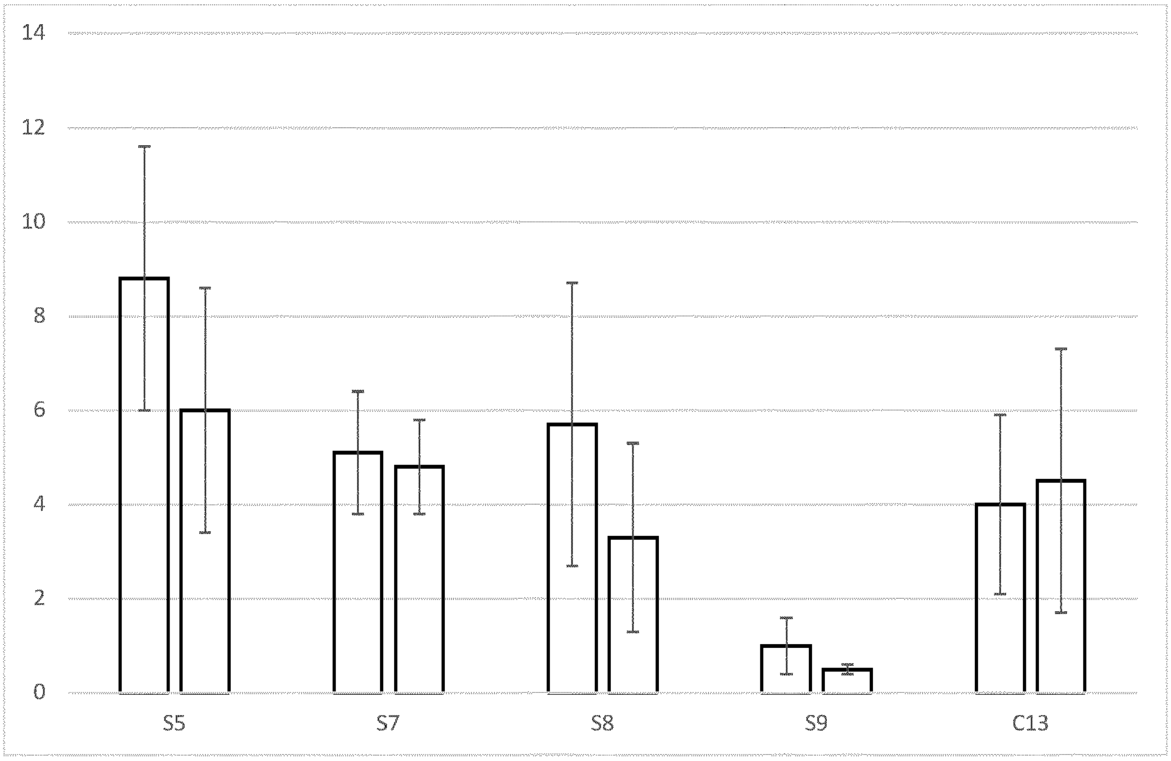


FIG. 29

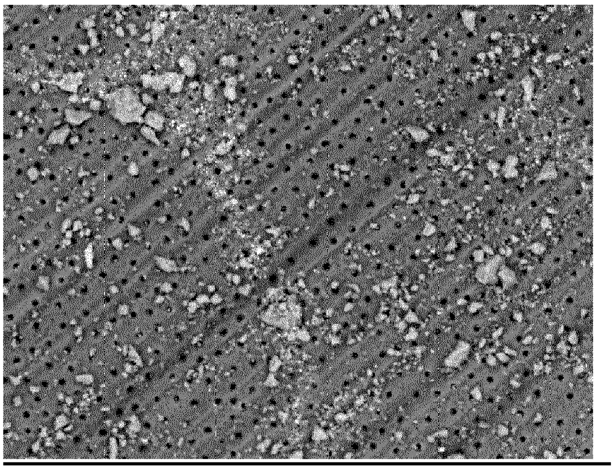

FIG. 30A

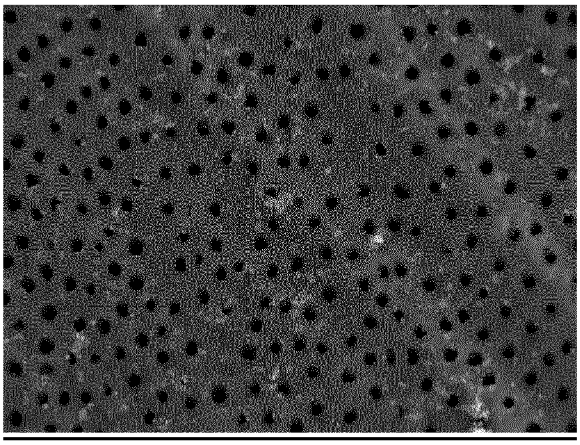

FIG. 30B

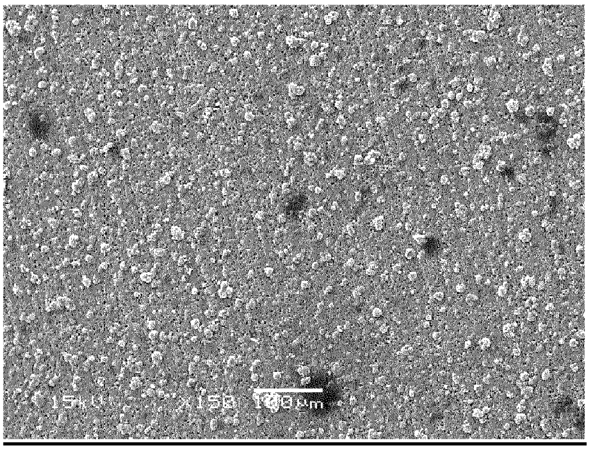


FIG. 31A

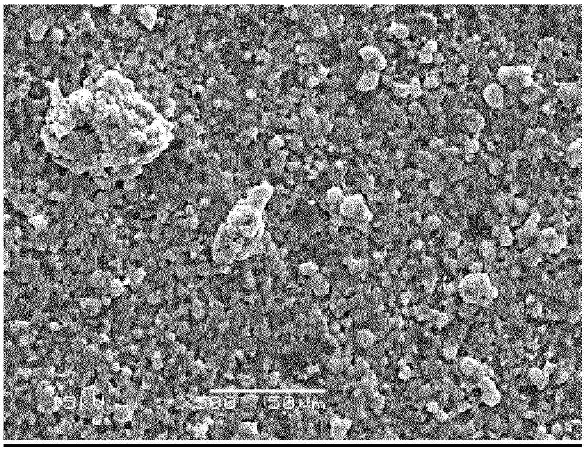


FIG. 31B

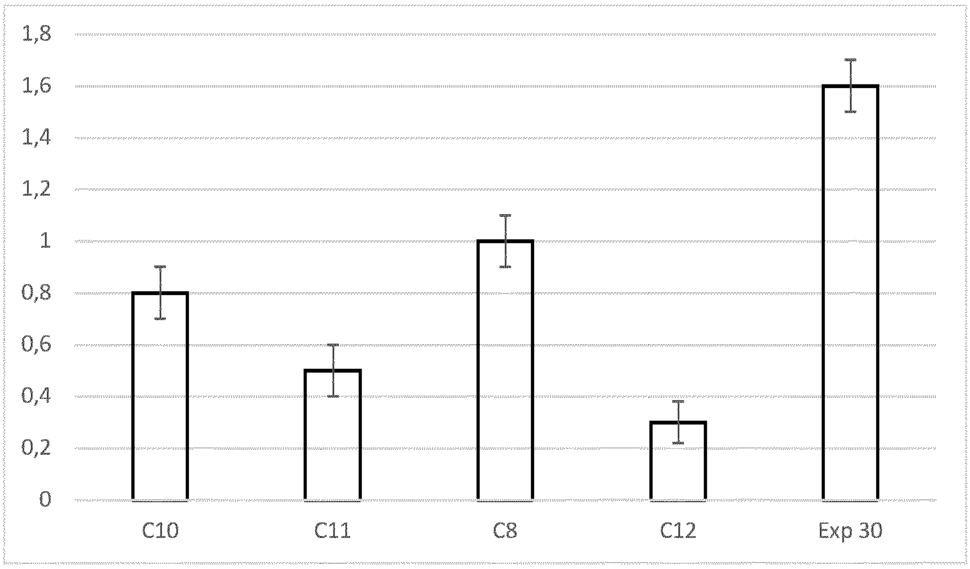


Fig. 32

Europäisches Patentamt
European Patent Office
Office européen des brevets

# EUROPEAN SEARCH REPORT

Application Number
EP 20 39 7518

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | QI YIPIN ET AL: "Effects of Molecular Weight and Concentration of Poly(Acrylic Acid) on Biomimetic Mineralization of Collagen", ACS BIOMATERIALS SCIENCE & ENGINEERING, vol. 4, no. 8, 13 August 2018 (2018-08-13), pages 2758-2766, XP055798103, US ISSN: 2373-9878, DOI: 10.1021/acsbiomaterials.8b00512 Retrieved from the Internet: URL:https://pubs.acs.org/doi/pdf/10.1021/acsbiomaterials.8b00512> * paragraphs [02.2], [03.1]; figures 1-4 * ----- | 1-13 | INV. A61K6/75 A61K6/816 A61K6/871 A61K6/873 A61K6/887 C08L33/10 A61K6/40 A61K6/824 A61K6/838 |
| Y | US 2010/086618 A1 (PASHLEY DAVID HENRY [US] ET AL) 8 April 2010 (2010-04-08) * paragraphs [0017], [0045], [0154] - [0167]; figures 3a,3b; example 1 * ----- | 1-13 | |
| Y | US 2018/078465 A1 (CHEN LIANG [US] ET AL) 22 March 2018 (2018-03-22) * paragraphs [0019] - [0021]; example 1; table 1 * ----- | 4,8-10 | TECHNICAL FIELDS SEARCHED (IPC) A61K C09J C08L |
| Y | JP 2018 002570 A (SOFSERA CORP) 11 January 2018 (2018-01-11) * paragraphs [0014], [0032] - [0036], [0043], [0087], [0088]; example 1 * ----- | 6,8 | |
| A | WO 2017/205058 A1 (ESSENTIAL DENTAL SYSTEMS INC [US]) 30 November 2017 (2017-11-30) * paragraphs [0013], [0047], [0056] - [0058], [0069]; example 1 * ----- -/-- | 1-13 | |

The present search report has been drawn up for all claims

1

| Place of search | Date of completion of the search | Examiner |
|---|---|---|
| The Hague | 30 April 2021 | Barenbrug-van Druten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 39 7518

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 108 324 578 B (UNIV WUHAN) 14 February 2020 (2020-02-14) * paragraphs [0004] - [0014]; example 1 * ----- | 1-13 | |
| A | CN 105 267 046 B (UNIV ZHEJIANG) 30 March 2018 (2018-03-30) * paragraphs [0024] - [0025]; example 1 * ----- | 1-13 | |

TECHNICAL FIELDS SEARCHED (IPC)

The present search report has been drawn up for all claims

1

| Place of search | Date of completion of the search | Examiner |
|---|---|---|
| The Hague | 30 April 2021 | Barenbrug-van Druten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

# ANNEX TO THE EUROPEAN SEARCH REPORT ON EUROPEAN PATENT APPLICATION NO. EP 20 39 7518

This annex lists the patent family members relating to the patent documents cited in the above-mentioned European search report. The members are as contained in the European Patent Office EDP file on The European Patent Office is in no way liable for these particulars which are merely given for the purpose of information.

30-04-2021

| Patent document cited in search report | Publication date | Patent family member(s) | Publication date |
|---|---|---|---|
| US 2010086618 A1 | 08-04-2010 | US 2010086618 A1 | 08-04-2010 |
| | | WO 2010042754 A2 | 15-04-2010 |
| US 2018078465 A1 | 22-03-2018 | CA 3037149 A1 | 29-03-2018 |
| | | EP 3515986 A1 | 31-07-2019 |
| | | US 2018078465 A1 | 22-03-2018 |
| | | WO 2018057353 A1 | 29-03-2018 |
| JP 2018002570 A | 11-01-2018 | JP 6072968 B1 | 01-02-2017 |
| | | JP 2018002570 A | 11-01-2018 |
| WO 2017205058 A1 | 30-11-2017 | CA 3026418 A1 | 30-11-2017 |
| | | EP 3463256 A1 | 10-04-2019 |
| | | US 2017340523 A1 | 30-11-2017 |
| | | WO 2017205058 A1 | 30-11-2017 |
| CN 108324578 B | 14-02-2020 | NONE | |
| CN 105267046 B | 30-03-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020035321 A


### Non-patent literature cited in the description


- **FERRACANE et al.** Academy of Dental Materials guidance-Resin composites: Part II-Technique sensitivity (handling, polymerization, dimensional changes). *Dental Materials,* 2017, vol. 33, 1171-1191
- *CHEMICAL ABSTRACTS,* 7732-18-5
- *CHEMICAL ABSTRACTS,* 10043-52-4